# EUROPEAN PATENT SPECIFICATION

(11) **EP 4 489 675 B1**
(45) Date of publication and mention of the grant of the patent: **07.05.2025**
(21) Application number: 24710508.3
(22) Date of filing: 28.02.2024
(51) Int. Cl.: A61B 18/14

(54) **THERMALLY AND MECHANICALLY SYMMETRIC ELECTRODES FOR END EFFECTORS**
THERMISCH UND MECHANISCH SYMMETRISCHE ELEKTRODEN FÜR ENDEFFEKTOREN
ÉLECTRODES À SYMÉTRIE THERMIQUE ET MÉCANIQUE POUR ORGANES TERMINAUX EFFECTEURS

(30) Priority: 01.03.2023 US 202318176984; 01.03.2023 US 202318177017; 01.03.2023 US 202318177035
(43) Date of publication of application: 15.01.2025
(73) Proprietor: Cilag GmbH International, 6300 Zug (CH)
(72) Inventor: DICKERSON, Benjamin D., Blue Ash, Ohio 45242 (US); HILL, Jason A., Blue Ash, Ohio 45242 (US); FISCHER, Austin Michael, Blue Ash, Ohio 45242 (US)
(74) Representative: Carpmaels & Ransford LLP
(86) International application number: PCT/IB2024/051912
(87) International publication number: WO 2024/180495

(56) References cited:
- WO-A2-2020/192847
- US-A1- 2015 297 288
- US-A1- 2017 202 608
- US-A1- 2020 305 965
- US-A1- 2022 226 050

## Description

### BACKGROUND

Minimally invasive surgical (MIS) instruments are often preferred over traditional open surgical devices due to reduced post-operative recovery time and minimal scarring. Laparoscopic surgery is one type of MIS procedure in which one or more small incisions are formed in the abdomen of a patient and a trocar is inserted through the incision to form a pathway that provides access to the abdominal cavity. Through the trocar, a variety of instruments and surgical tools can be introduced into the abdominal cavity. The instruments and tools introduced into the abdominal cavity via the trocar can be used to engage and/or treat tissue in a number of ways to achieve a diagnostic or therapeutic effect.

Various robotic systems have recently been developed to assist in MIS procedures. Robotic systems can allow for more instinctive hand movements by maintaining natural eye-hand axis. Robotic systems can also allow for more degrees of freedom in movement by including an articulable "wrist" joint that creates a more natural hand-like articulation. In such systems, an end effector positioned at the distal end of the instrument can be articulated (moved) using a cable driven motion system having one or more drive cables that extend through the wrist joint. A user (e.g., a surgeon) is able to remotely operate the end effector by grasping and manipulating in space one or more controllers that communicate with a tool driver coupled to the surgical instrument. User inputs are processed by a computer system incorporated into the robotic surgical system, and the tool driver responds by actuating the cable driven motion system. Moving the drive cables articulates the end effector to desired angular positions and configurations.

One type of end effector is a combination vessel sealer and tissue grasper that has opposing jaws capable of closing down and "grasping" onto tissue. Once tissue is properly grasped, a knife can be advanced distally within a knife slot to transect the grasped tissue, and electrical energy may be applied (prior to, during, or after transection) to the end effector to seal and cauterize the transected tissue.

It is desirable to improve vessel sealers and their operation to make minimally invasive surgeries more effective and efficient.

US 2015/297288 A1 relates to end-effector assemblies for use in surgical instruments and methods of manufacturing a pair of jaw members of an end-effector assembly.

### SUMMARY OF THE INVENTION

The present invention is defined in independent claim 1. Preferred embodiments are defined in the dependent claims.

### BRIEF DESCRIPTION OF THE DRAWINGS

The following figures are included to illustrate certain aspects of the present disclosure, and should not be viewed as exclusive embodiments. The subject matter disclosed is capable of considerable modifications, alterations, combinations, and equivalents in form and function, without departing from the scope of this disclosure.
FIG. 1 is a block diagram of an example robotic surgical system that may incorporate some or all of the principles of the present disclosure.
FIG. 2 is an isometric side view of an example surgical tool that may incorporate some or all of the principles of the present disclosure.
FIG. 3 illustrates potential degrees of freedom in which the wrist of the surgical tool of FIG. 2 may be able to articulate (pivot) and translate.
FIG. 4A is an enlarged isometric view of the distal end of the surgical tool of FIG. 2.
FIG. 4B an enlarged isometric view of the end effector of FIG. 4A, with one of the jaws removed.
FIGS. 5A and 5B are partial cross-sectional isometric and end views, respectively, of the end effector of FIGS. 4A-4B as taken along the plane indicated in FIG. 4A, according to one or more embodiments.
FIG. 6A is an isometric view of a portion of the lower jaw of the end effector of FIG. 2, according to one or more additional embodiments.
FIG. 6B is a cross-sectional view of the portion of the lower jaw of FIG. 6A, as taken along the indicated plane in FIG. 6A.
FIGS. 7A and 7B are side and isometric views, respectively, of the upper and lower electrodes, according to one or more embodiments.
FIGS. 8A and 8B are an isometric, partially exploded views of the end effector and the wrist of FIGS. 2 and 4A-4B, as taken from right and left vantage points, respectively.
FIGS. 9A and 9B are additional isometric, exploded views of the end effector and the wrist of FIGS. 2 and 4A-4B from right and left vantage points, respectively.
FIGS. 10A and 10B, illustrated are isometric exploded views of the end effector from left and right vantage points, respectively, according to one or more embodiments.
FIGS. 11A-11B are cross-sectional side views of the end effector in the closed and open positions, respectively, according to one or more embodiments.
FIG. 11C is a schematic side view of the end effector and the wrist, according to one or more embodiments.
FIGS. 12A and 12B are exploded, isometric views of the distal and proximal clevises of the wrist, as taken from right and left vantage points, according to one or more embodiments.
FIG. 13A is an enlarged side view of the wrist of FIGS. 12A-12B, according to one or more embodiments.
FIG. 13B is a cross-sectional top view of the wrist of FIGS. 12A-12B, according to one or more embodiments.
FIG. 14A is another enlarged end view of the end effector, according to one or more additional embodiments.
FIG. 14B is an isometric top view of the lower jaw, according to one or more embodiments.
FIGS. 15A and 15B are enlarged isometric views of the knife and the knife housing, according to one or more embodiments.
FIG. 16 is an enlarged cross-sectional side view of portions of the end effector and the wrist, according to one or more embodiments.
FIG. 17 is a cross-sectional end view of the wrist as taken through the first pivot axis, according to one or more embodiments.
FIG. 18 is a schematic flowchart of an example process algorithm, according to one or more embodiments of the present disclosure.

### DETAILED DESCRIPTION

The present disclosure is related to robotic surgical systems and, more particularly, to improved designs for tissue grasper end effectors.

One example end effector includes a first jaw having a first electrode and a first insulator secured thereto, and a second jaw having a second electrode and a second insulator secured thereto, the first and second jaws being pivotable between open and closed positions. A knife slot may be cooperatively defined in the first and second jaws and define a diamond-shape cross-section when the first and second jaws are in the closed position. A knife may be extendable into the knife slot and longitudinally movable within the knife slot.

A surgical tool disclosed herein includes a wrist including a proximal clevis and a distal clevis rotatably mountable to the proximal clevis at a first pivot axis, and an end effector rotatably coupled to the distal clevis at a second pivot axis, the end effector including opposing first and second jaws rotatably coupled to each other at a jaw pivot axis. The jaw pivot axis may be parallel to the second pivot axis and orthogonal to the first pivot axis, and a longitudinal axis of the end effector may be perpendicular to and intersects the jaw pivot axis.

Another example end effector includes opposing first and second jaws rotatably coupled to each other at a jaw pivot axis, a knife slot defined in one or both of the first and second jaws, and a knife coupled to a distal end of a drive rod and longitudinally extendable into the knife slot. A knife housing may be pivotably coupled between the first and second jaws at the jaw pivot axis and define a cavity sized to receive the knife. The drive rod may be actuatable to move the knife between a stowed position, where the knife is received within the cavity, and an extended position, where the knife is extended out of the cavity and into the knife slot.

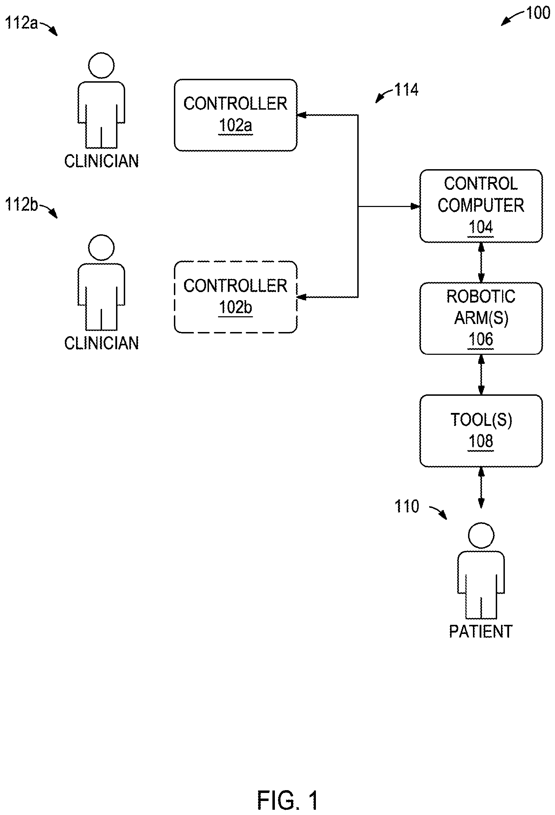

FIG. 1 is a block diagram of an example robotic surgical system 100 that may incorporate some or all of the principles of the present disclosure. As illustrated, the system 100 can include at least one set of user input controllers 102a and at least one control computer 104. The control computer 104 may be mechanically and/or electrically coupled to a robotic manipulator and, more particularly, to one or more robotic arms 106 (alternately referred to as "tool drivers"). In some embodiments, the robotic manipulator may be included in or otherwise mounted to an arm cart capable of making the system portable. Each robotic arm 106 may include and otherwise provide a location for mounting one or more surgical instruments or tools 108 for performing various surgical tasks on a patient 110. Operation of the robotic arms 106 and associated tools 108 may be directed by a clinician 112a (e.g., a surgeon) from the user input controller 102a.

In some embodiments, a second set of user input controllers 102b (shown in dashed line) may be operated by a second clinician 112b to direct operation of the robotic arms 106 and tools 108 via the control computer 104 and in conjunction with the first clinician 112a. In such embodiments, for example, each clinician 112a,b may control different robotic arms 106 or, in some cases, complete control of the robotic arms 106 may be passed between the clinicians 112a,b as needed. In some embodiments, additional robotic manipulators having additional robotic arms may be utilized during surgery on the patient 110, and these additional robotic arms may be controlled by one or more of the user input controllers 102a,b.

The control computer 104 and the user input controllers 102a,b may be in communication with one another via a communications link 114, which may be any type of wired or wireless telecommunications means configured to carry a variety of communication signals (e.g., electrical, optical, infrared, etc.) according to any communications protocol. In some applications, for example, there is a tower with ancillary equipment and processing cores designed to drive the robotic arms 106.

The user input controllers 102a,b generally include one or more physical controllers that can be grasped by the clinicians 112a,b and manipulated in space while the surgeon views the procedure via a stereo display. The physical controllers generally comprise manual input devices movable in multiple degrees of freedom, and which often include an actuatable handle for actuating the surgical tool(s) 108, for example, for opening and closing opposing jaws, applying an electrical potential (current) to an electrode, or the like. The control computer 104 can also include an optional feedback meter viewable by the clinicians 112a,b via a display to provide a visual indication of various surgical instrument metrics, such as the amount of force being applied to the surgical instrument (i.e., a cutting instrument or dynamic clamping member).

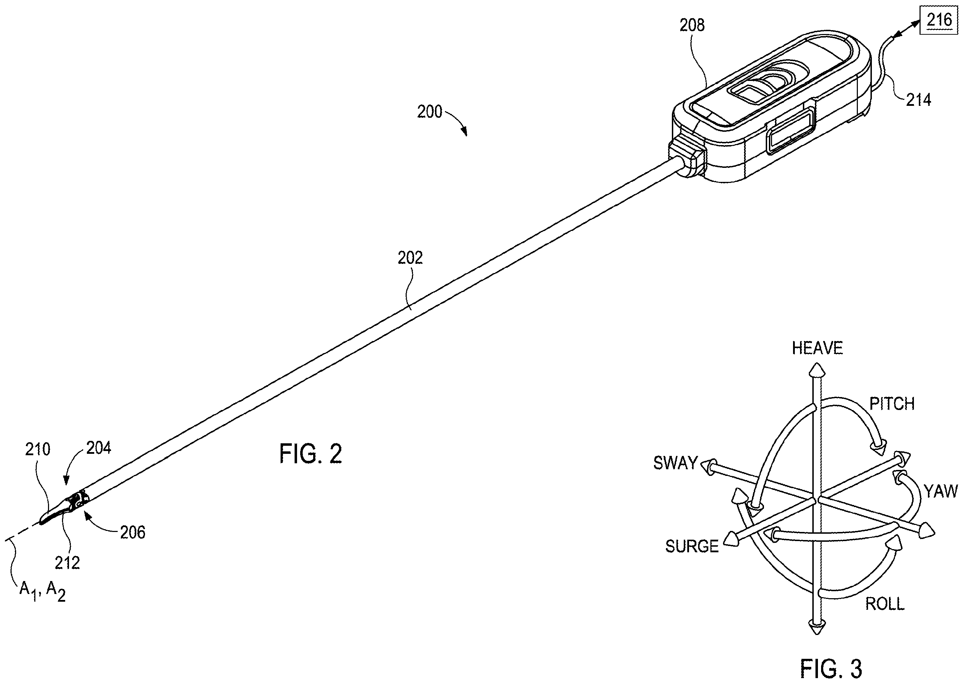

FIG. 2 is an isometric side view of an example surgical tool 200 that may incorporate some or all of the principles of the present disclosure. The surgical tool 200 may be the same as or similar to the surgical tool(s) 108 of FIG. 1 and, therefore, may be used in conjunction with a robotic surgical system, such as the robotic surgical system 100 of FIG. 1. Accordingly, the surgical tool 200 may be designed to be releasably coupled to a tool driver included in the robotic surgical system 100. In other embodiments, however, aspects of the surgical tool 200 may be adapted for use in a manual or hand-operated manner, without departing from the scope of the disclosure.

As illustrated, the surgical tool 200 includes an elongated shaft 202, an end effector 204, a wrist 206 (alternately referred to as a "wrist joint" or an "articulable wrist joint") that couples the end effector 204 to the distal end of the shaft 202, and a drive housing 208 coupled to the proximal end of the shaft 202. In applications where the surgical tool is used in conjunction with a robotic surgical system (e.g., the robotic surgical system 100 of FIG. 1), the drive housing 208 can include coupling features that releasably couple the surgical tool 200 to the robotic surgical system.

The terms "proximal" and "distal" are defined herein relative to a robotic surgical system having an interface configured to mechanically and electrically couple the surgical tool 200 (e.g., the housing 208) to a robotic manipulator. The term "proximal" refers to the position of an element closer to the robotic manipulator and the term "distal" refers to the position of an element closer to the end effector 204 and thus further away from the robotic manipulator. Alternatively, in manual or hand-operated applications, the terms "proximal" and "distal" are defined herein relative to a user, such as a surgeon or clinician. The term "proximal" refers to the position of an element closer to the user and the term "distal" refers to the position of an element closer to the end effector 204 and thus further away from the user. Moreover, the use of directional terms such as above, below, upper, lower, upward, downward, left, right, and the like are used in relation to the illustrative embodiments as they are depicted in the figures, the upward or upper direction being toward the top of the corresponding figure and the downward or lower direction being toward the bottom of the corresponding figure.

During use of the surgical tool 200, the end effector 204 is configured to move (pivot) relative to the shaft 202 at the wrist 206 to position the end effector 204 at desired orientations and locations relative to a surgical site. To accomplish this, the housing 208 includes (contains) various drive inputs and mechanisms (e.g., gears, actuators, etc.) designed to control operation of various features associated with the end effector 204 (e.g., clamping, firing, cutting, rotation, articulation, etc.). In at least some embodiments, the shaft 202, and hence the end effector 204 coupled thereto, is configured to rotate about a longitudinal axis A₁ of the shaft 202. In such embodiments, at least one of the drive inputs included in the housing 208 is configured to control rotational movement of the shaft 202 about the longitudinal axis A₁.

The shaft 202 is an elongate member extending distally from the housing 208 and has at least one lumen extending therethrough along its axial length. In some embodiments, the shaft 202 may be fixed to the housing 208, but could alternatively be rotatably mounted to the housing 208 to allow the shaft 202 to rotate about the longitudinal axis A₁. In yet other embodiments, the shaft 202 may be releasably coupled to the housing 208, which may allow a single housing 208 to be adaptable to various shafts having different end effectors.

The end effector 204 can exhibit a variety of sizes, shapes, and configurations. In the illustrated embodiment, the end effector 204 comprises a combination tissue grasper and vessel sealer that include opposing jaws 210, 212 configured to move (articulate) between open and closed positions. As will be appreciated, however, the opposing jaws 210, 212 may alternatively form part of other types of end effectors such as, but not limited to, a surgical scissors, a clip applier, a needle driver, a babcock including a pair of opposed grasping jaws, bipolar jaws (e.g., bipolar Maryland grasper, forceps, a fenestrated grasper, etc.), etc. One or both of the jaws 210, 212 may be configured to pivot to articulate the end effector 204 between the open and closed positions.

FIG. 3 illustrates the potential degrees of freedom in which the wrist 206 may be able to articulate (pivot). The wrist 206 can have any of a variety of configurations. In general, the wrist 206 comprises a joint configured to allow pivoting movement of the end effector 204 relative to the shaft 202. The degrees of freedom of the wrist 206 are represented by three translational variables (i.e., surge, heave, and sway), and by three rotational variables (i.e., Euler angles or roll, pitch, and yaw). The translational and rotational variables describe the position and orientation of the end effector 204 with respect to a given reference Cartesian frame. As depicted in FIG. 3, "surge" refers to forward and backward translational movement, "heave" refers to translational movement up and down, and "sway" refers to translational movement left and right. With regard to the rotational terms, "roll" refers to tilting side to side, "pitch" refers to tilting forward and backward, and "yaw" refers to turning left and right.

The pivoting motion can include pitch movement about a first axis of the wrist 206 (e.g., X-axis), yaw movement about a second axis of the wrist 206 (e.g., Y-axis), and combinations thereof to allow for 360° rotational movement of the end effector 204 about the wrist 206. In other applications, the pivoting motion can be limited to movement in a single plane, e.g., only pitch movement about the first axis of the wrist 206 or only yaw movement about the second axis of the wrist 206, such that the end effector 204 moves only in a single plane.

Referring again to FIG. 2, the surgical tool 200 may also include a plurality of drive cables (obscured in FIG. 2) that form part of a cable driven motion system configured to facilitate movement and articulation of the end effector 204 relative to the shaft 202. Moving (actuating) one or more of the drive cables moves the end effector 204 between an unarticulated position and an articulated position. The end effector 204 is depicted in FIG. 2 in the unarticulated position where a longitudinal axis A₂ of the end effector 204 is substantially aligned with the longitudinal axis A₁ of the shaft 202, such that the end effector 204 is at a substantially zero angle relative to the shaft 202. Due to factors such as manufacturing tolerance and precision of measurement devices, the end effector 204 may not be at a precise zero angle relative to the shaft 202 in the unarticulated position, but nevertheless be considered "substantially aligned" thereto. In the articulated position, the longitudinal axes A₁, A₂ would be angularly offset from each other such that the end effector 204 is at a non-zero angle relative to the shaft 202.

In some embodiments, the surgical tool 200 may be supplied with electrical power (current) via a power cable 214 coupled to the housing 208. In other embodiments, the power cable 214 may be omitted and electrical power may be supplied to the surgical tool 200 via an internal power source, such as one or more batteries or fuel cells. In such embodiments, the surgical tool 200 may alternatively be characterized and otherwise referred to as an "electrosurgical instrument" capable of providing electrical energy to the end effector 204.

The power cable 214 may place the surgical tool 200 in electrical communication with a generator 216 that supplies energy, such as electrical energy (e.g., radio frequency energy), ultrasonic energy, microwave energy, heat energy, or any combination thereof, to the surgical tool 200 and, more particularly, to the end effector 204. Accordingly, the generator 216 may comprise a radio frequency (RF) source, an ultrasonic source, a direct current source, and/or any other suitable type of electrical energy source that may be activated independently or simultaneously.

In applications where the surgical tool 200 is configured for bipolar operation, the power cable 214 will include a supply conductor and a return conductor. Current can be supplied from the generator 216 to an active (or source) electrode located at the end effector 204 via the supply conductor, and current can flow back to the generator 216 via a return electrode located at the end effector 204 via the return conductor. In the case of a bipolar grasper with opposing jaws, for example, the jaws serve as the electrodes where the proximal end of the jaws are isolated from one another and the inner surface of the jaws (i.e., the area of the jaws that grasp tissue) apply the current in a controlled path through the tissue. In applications where the surgical tool 200 is configured for monopolar operation, the generator 216 transmits current through a supply conductor to an active electrode located at the end effector 204, and current is returned (dissipated) through a return electrode (e.g., a grounding pad) separately coupled to a patient's body.

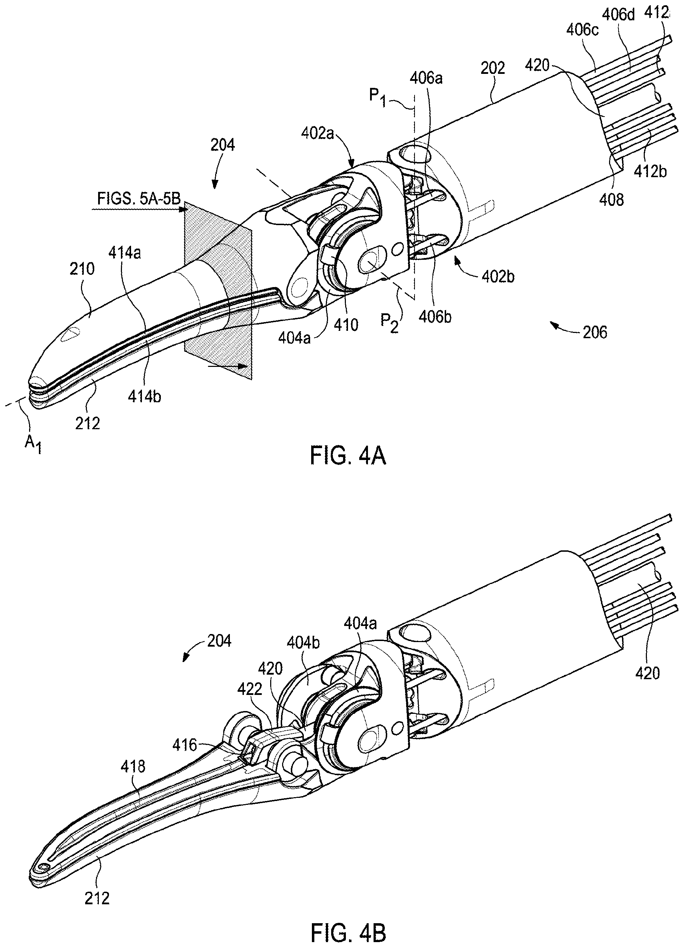

FIG. 4A is an enlarged isometric view of the distal end of the surgical tool 200. More specifically, FIG. 4A depicts an enlarged view of the end effector 204 and the wrist 206, with the jaws 210, 212 of the end effector 204 in the closed position. The wrist 206 operatively couples the end effector 204 to the shaft 202. In some embodiments, however, a shaft adapter may be directly coupled to the wrist 206 and otherwise interpose the shaft 202 and the wrist 206. Accordingly, the wrist 206 may be operatively coupled to the shaft 202 either through a direct coupling engagement where the wrist 206 is directly coupled to the distal end of the shaft 202, or an indirect coupling engagement where a shaft adapter interposes the wrist 206 and the distal end of the shaft 202. As used herein, the term "operatively couple" refers to a direct or indirect coupling engagement between two components.

To operatively couple the end effector 204 to the shaft 202, the wrist 206 includes a first or "distal" clevis 402a and a second or "proximal" clevis 402b. The clevises 402a,b may alternatively be referred to as "articulation joints" or "linkages." As described herein, the clevises 402a,b are operatively coupled to facilitate articulation of the wrist 206 relative to the shaft 202, thereby allowing the end effector 204 to articulate in yaw, pitch, or a combination of both yaw and pitch.

As illustrated, the proximal end of the distal clevis 402a may be rotatably mounted to the distal end of the proximal clevis 402b at a first pivot axis P₁ of the wrist 206. First and second pulleys 404a and 404b (only the first pulley 404a is visible in FIG. 4A; see FIG. 4B) may be rotatably mounted to the distal end of the distal clevis 402a at a second pivot axis P₂ of the wrist 206. The first pivot axis P₁ is substantially perpendicular (orthogonal) to the longitudinal axis A₁ of the shaft 202, and the second pivot axis P₂ is substantially perpendicular (orthogonal) to both the longitudinal axis A₁ and the first pivot axis P₁. Movement of the end effector 204 about the first pivot axis P₁ provides "yaw" articulation of the wrist 206, and movement about the second pivot axis P₂ provides "pitch" articulation of the wrist 206.

A plurality of drive members, shown as drive members 406a, 406b, 406c, and 406d, extend longitudinally within a lumen 408 defined by the shaft 202 (or a shaft adaptor) and extend at least partially through the wrist 206. The drive members 406a-d may form part of the actuation systems housed within the drive housing 208 (FIG. 2), and may comprise cables, bands, lines, cords, wires, woven wires, ropes, strings, twisted strings, elongate members, belts, shafts, flexible shafts, drive rods, or any combination thereof. The drive members 406a-d can be made from a variety of materials including, but not limited to, a metal (e.g., tungsten, stainless steel, nitinol, etc.) a polymer (e.g., ultra-high molecular weight polyethylene), a synthetic fiber (e.g., KEVLAR^{®}, VECTRAN^{®}), etc.), an elastomer, or any combination thereof. While four drive members 406a-d are depicted in FIG. 4A, more or less than four may be employed, without departing from the scope of the disclosure.

The drive members 406a-d extend proximally from the end effector 204 and the wrist 206 toward the drive housing 208 (FIG. 2) where they are operatively coupled to various actuation mechanisms or devices that facilitate longitudinal movement (translation) of the drive members 406a-d within the lumen 408. Selective actuation of the drive members 406a-d applies tension (i.e., pull force) to the given drive member 406a-d in the proximal direction, which urges the given drive member 406a-d to translate longitudinally within the lumen 408.

In the illustrated embodiment, the drive members 406a-d each extend longitudinally through the proximal clevis 402b, and the distal end of each drive member 406a-d terminates at the first or second pulleys 404a,b, thus operatively coupling each drive member 406a-d to the end effector 204. In some embodiments, the distal ends of the first and second drive members 406a,b may be coupled to each other and terminate at the first pulley 404a, and the distal ends of the third and fourth drive members 406c,d may be coupled to each other and terminate at the second pulley 404b (FIG. 4B). In at least one embodiment, the distal ends of the first and second drive members 406a,b and the distal ends of the third and fourth drive members 406c,d may each be coupled together at a corresponding ball crimp 410 (only one shown) mounted to the first or second pulley 404a,b, respectively.

In the illustrated embodiment, the drive members 406a-d operate "antagonistically". More specifically, when the first drive member 406a is actuated (moved in tension), the second drive member 406b naturally follows as coupled to the first drive member 406a, and vice versa. Similarly, when the third drive member 406c is actuated (moved in tension), the fourth drive member 406d naturally follows as coupled to the third drive member 406c, and vice versa. Antagonistic operation of the drive members 406a-d can open or close the jaws 210, 212 and can further cause the end effector 204 to articulate at the wrist 206. More specifically, selective actuation of the drive members 406a-d in known configurations or coordination can cause the end effector 204 to articulate about one or both of the pivot axes P₁, P₂, thus facilitating articulation of the end effector 204 in both pitch and yaw directions. Moreover, selective actuation of the drive members 406a-d in other known configurations or coordination will cause the jaws 210, 212 to open or close. Antagonistic operation of the drive members 406a-d advantageously reduces the number of cables required to provide full wrist 206 motion, and also helps eliminate slack in the drive members 406a-d, which results in more precise motion of the end effector 204.

In the illustrated embodiment, the end effector 204 is able to articulate (move) in pitch about the second or "pitch" pivot axis P₂, which is located near the distal end of the wrist 206. Thus, the jaws 210, 212 open and close in the direction of pitch. Moving both articulation axes P₁, P₂ closer to the therapeutic jaw surface enables minimization of the distance between the remote center of motion, therapeutic surface, and articulation axis. Having the pitch pivot axis P₂ as far distal as possible may be advantageous in providing a geometric advantage that helps an operator more easily get under vessels and facilitate blunt (touch) and spread dissection. This may also reduce the overall length of the end effector 204 and thereby improve surgeon access to patient anatomy during surgery by allowing discrete motion in smaller surgical spaces. This may also improve the robotic control of the instrument making user-applied motions seem more natural. This may further result in providing better reach to anatomy during dissection, such as for lymph node removal or other tissue mobilization. In other embodiments, however, the wrist 206 may alternatively be configured such that the second pivot axis P₂ facilitates yaw articulation of the jaws 210, 212, without departing from the scope of the disclosure.

In the illustrated embodiment, first and second electrical conductors 412a and 412b also extend longitudinally within the lumen 408, through the wrist 206, and terminate at the end effector 204 to supply electrical energy thereto. More particularly, the first electrical conductor 412a terminates at a first or "upper" electrode 414a secured to the upper jaw 210, and the second electrical conductor 412b terminates at a second or "lower" electrode 414b secured to the lower jaw 212. In some embodiments, the electrical conductors 412a,b may each comprise a wire, but may alternatively comprise a rigid or semi-rigid shaft, rod, or strip (ribbon) made of a conductive material. The electrical conductors 412a,b may be partially covered with an insulative covering (overmold) made of a non-conductive material. Routing the electrical conductors 412a,b to the corresponding electrodes 414a,b, respectively, allows the end effector 204 to operate in bipolar RF operation.

In at least one embodiment, the electrical energy conducted through the electrical conductors 412a,b exhibits a frequency between about 100 kHz and 1 MHz. In a process known as Joule heating (resistive or Ohmic heating) the RF energy is transformed into heat within target tissue grasped between the jaws 210, 212 due the tissue's intrinsic electrical impedance, thereby increasing the temperature of the target tissue. Heating the target tissue achieves various tissue effects such as cauterization and/or coagulation, and thus may be particularly useful for sealing blood vessels or diffusing bleeding during a surgical procedure. Heating the target tissue may also cause desiccation of the tissue, which allows the tissue to be cut (dissected) more easily.

FIG. 4B is another enlarged isometric view of the end effector 204. The upper jaw 210 (FIG. 4A) is omitted in FIG. 4B to enable viewing of various component parts of the lower jaw 212. In the illustrated embodiment, the end effector 204 comprises a combination tissue grasper and vessel sealer and includes a knife 416 (mostly occluded), alternately referred to as a "cutting element" or "blade." The knife 416 is aligned with and configured to traverse a guide track or "knife slot" 418 defined longitudinally in both the upper and lower jaws 210, 212. The knife 416 may be operatively coupled to the distal end of a drive rod 420 (alternately referred to as "knife rod," "actuation rod," or "push rod") that extends longitudinally within the lumen 408 and passes through the wrist 206. Longitudinal movement (translation) of the drive rod 420 correspondingly moves the knife 416 within the knife slot 418.

The drive rod 420 may comprise a rigid or semi rigid elongate member, such as a rod or shaft (e.g., a hypotube, a hollow rod, a solid rod, etc.), a wire, a ribbon, a push cable, or any combination thereof. The drive rod 420 can be made from a variety of materials including, but not limited to, metal (e.g., tungsten, nitinol, stainless steel, etc.), a polymer, or a composite material. The drive rod 420 may have a circular cross-section, but may alternatively exhibit a polygonal cross-section without departing from the scope of the disclosure.

Similar to the drive members 406a-d, the drive rod 420 may form part of the actuation systems housed within the drive housing 208 (FIG. 2). Selective actuation of a corresponding drive input will cause the drive rod 420 to move distally or proximally within the lumen 408, and correspondingly move the knife 416 in the same longitudinal direction.

In the illustrated embodiment, the knife 416 is shown received within a knife housing 422 pivotably mounted to the end effector 204. As described in more detail below, the knife housing 422 defines a central passageway through which the knife 416 and the drive rod 420 are able to extend to move the knife 416 into and along the knife slot 418. Upon firing the end effector 204, the drive rod 420 is moved (urged) distally, which correspondingly moves the knife 416 out of the knife housing 422 and into the knife slot 418. After firing is complete, the drive rod 420 is retracted proximally, which pulls the knife 416 proximally and back into the knife housing 422 until it is desired to again fire the end effector 204.

### Thermally and Mechanically Symmetric Electrodes

Referring again to FIG. 4A, with continued reference to FIG. 4B, in example operation of the end effector 204, the jaws 210, 212 may be actuated to close and grasp onto tissue, following which the electrodes 414a,b may be supplied with electrical energy, which is transformed into heat within the grasped tissue to cauterize, coagulate, and/or otherwise seal the tissue. The knife 416 may then be advanced distally along the knife slot 418 to cut (transect) and simultaneously seal the grasped tissue. Alternatively, in other applications, the knife 416 may be advanced prior to the application of electrical energy to cut unsealed tissue to facilitate dissection of non-vascular tissue.

According to embodiments of the present disclosure, the electrodes 414a,b provided in the end effector 204 may be designed to be thermally and mechanically symmetric and thereby configured to optimize performance of the end effector 204 when treating and cutting tissue grasped between the jaws 210, 212. Prior vessel sealer designs commonly include only a single, isolated electrode positioned on only one of the jaws, and the opposing jaw operates as the return path on the opposite side. This design configuration results in a difference in thermal mass causing asymmetric heat signatures on opposing sides of the tissue grasped between the jaws, which may affect tissue sealing performance. In contrast, the thermally and mechanically symmetric jaws 210, 212 described herein each include a corresponding powered electrode 414a,b, which results in a thermally balanced thermal mass on both sides (e.g., above and below) of the grasped tissue. The presently-disclosed electrodes 414a,b facilitate consistent and efficient heating of the electrodes 414a,b and the corresponding grasped tissues due to low and equal thermal mass on both sides of the tissue.

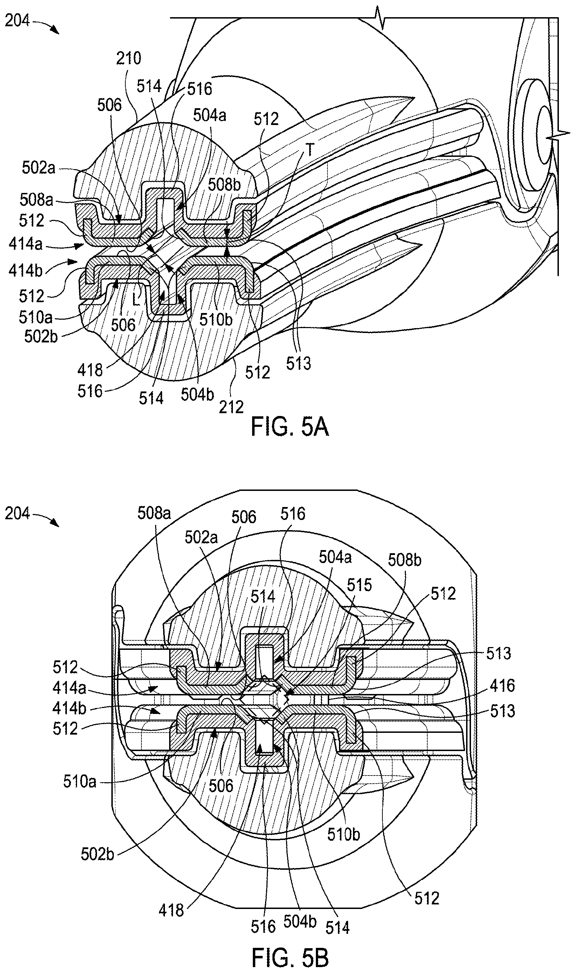

FIGS. 5A and 5B are partial cross-sectional isometric and end views, respectively, of the end effector 204, as taken along the plane indicated in FIG. 4A, according to one or more embodiments. The upper and lower jaws 210, 212 are shown in FIGS. 5A-5B in the closed position. In the closed position, the upper jaw 210 extends substantially parallel to the lower jaw 212 and the electrodes 414a,b interpose at least a portion of the interface between the upper and lower jaws 210, 212. Each electrode 414a,b is mounted to the corresponding jaw 210, 212 in conjunction with a corresponding insulator 502a and 502b, which operates to isolate the RF energy supplied to the electrodes 414a,b from the corresponding bodies of the jaws 210, 212 during operation.

As illustrated, the upper insulator 502a may be overmolded onto the upper electrode 414a, and the lower insulator 502b may be overmolded onto the lower electrode 414b. Each insulator 502a,b may comprise a non-conductive material mated or otherwise coupled to the corresponding jaw 210, 212. Suitable non-conductive materials include, but are not limited to, nylon, polyphthalamide (PPA; e.g., GRIVORY^{®} or THERMEC^{™}), or a combination thereof.

The electrodes 414a,b and the insulators 502a,b may cooperatively define the knife slot 418 that guides the knife 416 (FIGS. 4B and 5B) along the jaws 210, 212. More specifically, the upper insulator 502a and the upper electrode 414a may cooperatively define a first or "upper" slot portion 504a, and the lower insulator 502b and the lower electrode 414b may cooperatively define a second or "lower" slot portion 504b. When the jaws 210, 212 are closed, the upper and lower slot portions 504a,b are aligned such that the knife 416 extends partially into each slot portion 504a,b as the knife 416 traverses the knife slot 418. The slot portions 504a,b cooperatively direct the travel path of the knife 416 and help prevent the knife 416 from twisting or otherwise falling (leaning) to one side or the other.

The upper and lower electrodes 414a,b constitute mirror images of each other, and each electrode 414a,b provides and otherwise defines a planar sealing surface 506. When the jaws 210, 212 are closed, the planar sealing surfaces 506 are arranged substantially parallel to each other and a small gap is defined therebetween to receive (accommodate) tissue. The upper slot portion 504a bifurcates the planar sealing surface 506 of the upper electrode 414a, thereby defining left (first) and right (second) portions 508a and 508b of the upper electrode 414a. Similarly, the lower slot portion 504b bifurcates the planar sealing surface 506 of the lower electrode 414b, thereby defining left (first) and right (second) portions 510a and 510b of the lower electrode 414b.

The design and configuration of the lateral extents of the left and right portions 508a,b and 510a,b may be optimized for efficient thermal management. More specifically, the left and right portions 508a,b and 510a,b of each electrode 414a,b provide and otherwise define an outer lateral extent 512 extending from the corresponding planar sealing surface 506. In the illustrated embodiment, the outer lateral extent 512 (alternately referred to as the "perimeter" or "boundary") extends away from the planar sealing surface 506 and away from the opposing jaw 210, 212; e.g., out of the plane of the planar sealing surface 506 and toward the body of the corresponding jaw 210, 212. In at least one embodiment, as illustrated, the outer lateral extent 512 extends from the corresponding planar sealing surface 506 at about a 90° angle, but could alternatively extend at an angle greater or less than 90°, without departing from the scope of the disclosure.

In some embodiments, as illustrated, one or more of the outer lateral extents 512 of the electrodes 414a,b may be embedded within a portion of the corresponding insulator 502a,b. As will be appreciated, this may help retain (secure) the electrode 414a,b to the insulator 502a,b without requiring mechanical fasteners, adhesives, or an interference fit. Moreover, embedding the outer lateral extents 512 in the insulator 502a,b may also be advantageous in reducing the conductive pathway through tissue extending on either lateral side of the jaws 210, 212. More specifically, embedding at least a portion of the outer lateral extents 512 within the insulator 502a,b may result in cooler tissue protruding out each lateral side of the jaws 210, 212 as compared to end effectors with entirely exposed lateral extents.

In the illustrated embodiment, the insulator 502a,b extends toward but stops short of the planar sealing surface 506. As a result the electrodes 414a,b may provide an electrically exposed edge 513 (e.g., radius, curvature, etc.) that provides the transition between the planar sealing surface and the lateral extent 512. In other embodiments, however, and as described in more detail below, the insulator 502a,b may extend to and terminate at the planar sealing surface 506, thus covering and otherwise encapsulating the edge 513. In such embodiments, the heat difference in the portions of the tissue protruding out each lateral side of the jaws 210, 212 may be even cooler as compared to temperatures where the electrodes 414a,b directly contact the tissue during sealing.

The left and right portions 508a,b and 510a,b of each electrode 414a,b may further provide and otherwise define an inner lateral extent 514 extending from the corresponding planar sealing surface 506 at the knife slot 418. The inner lateral extent 514 could alternately be referred to as or characterized as an inner lateral "perimeter," "boundary," or "face". Similar to the outer lateral extents 512, each inner lateral extent 514 extends away from the planar sealing surface 506 and also away from the opposing jaw 210, 212; e.g., out of the plane of the planar sealing surface 506 and toward the body of the corresponding jaw 210, 212). In at least one embodiment, as illustrated, the inner lateral extent 514 extends away from the corresponding planar sealing surface 506 at about a 45° angle, but could alternatively extend at an angle greater or less than 45°, without departing from the scope of the disclosure.

One or more of the inner lateral extents 514 may be at least partially embedded within a portion of the corresponding insulator 502a,b. As will be appreciated, this may help retain (secure) the electrode 414a,b to the insulator 502a,b without the need of mechanical fasteners or an interference fit.

As forming integral parts of the corresponding upper and lower slot portions 504a,b, the inner lateral extents 514 help define the knife slot 418. In at least one embodiment, when the jaws 210, 212 are closed, the electrodes 414a,b at the inner lateral extents 514 cooperatively define a generally diamond-shaped cross-section 515 (FIG. 5B). The diamond-shaped cross-section 515 of the knife slot 418 may be sized to provide adequate space for the knife 416 (FIG. 5B) and the drive rod 420 (FIG. 4B) to extend therethrough while firing the end effector 204.

Moreover, the diamond-shape 515 of the electrodes 414a,b at the knife slot 418 is electrically exposed (e.g., not overmolded with the insulators 502a,b), which may provide a conductive pathway that creates uniform heating of tissue across the knife slot 418. This may create a thermal effect that helps desiccate tissue in the center of the knife slot 418, which makes the tissue easier to cut and ensures that the grasped tissue is fully sealed up to the cut location. Electrodes without the diamond-shaped cross-section 515 (i.e., entirely flat sealing surfaces) can fail to communicate sufficient thermal energy to the tissue at the knife slot. Having the diamond-shaped 515 knife slot 418, however, allows the thermal energy to radiate to and thereby efficiently desiccate the grasped tissue.

In some embodiments, one or both of the insulators 502a,b may provide or otherwise define a floor or "trough" section 516 extending laterally across the knife slot 418 and thereby structurally connecting the lateral sides of the corresponding insulators 502a,b. Each trough section 516 may form the bottom of the upper and lower slot portions 504a,b. Prior art insulators are often disconnected (separated) at the knife slot, but the insulators 502a,b described herein comprise monolithic components that extend across the knife slot 418 and opposing lateral sides are interconnected at the trough section 516. As will be appreciated, the trough section 516 may prove advantageous in simplifying the manufacture of the insulators 502a,b.

Referring specifically to FIG. 5A, the inner lateral extents 514 each provide an exposed surface area extending from the corresponding planar sealing surface 506 and toward the trough section 516, and thereby help form a portion of the knife slot 418. In some embodiments, as illustrated, the length L of one or both of the inner lateral extents 514 may be greater than a thickness T of the corresponding electrode 414a,b. This may prove advantageous in providing extra surface area for sealing through the knife zone.

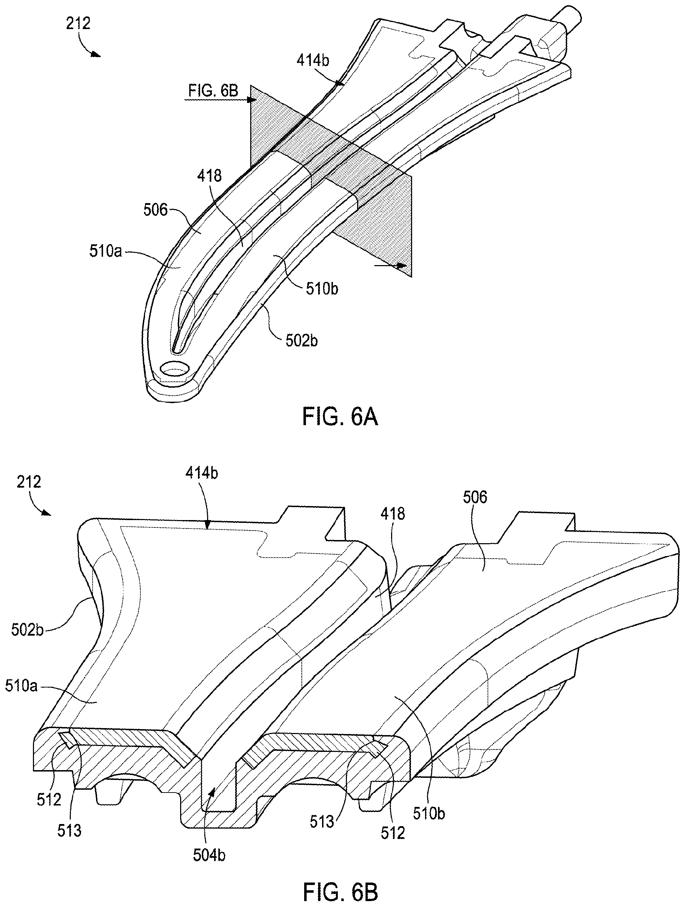

FIG. 6A is an isometric view of a portion of the lower jaw 212, according to one or more additional embodiments, and FIG. 6B is a cross-sectional view of the portion of the lower jaw 212 of FIG. 6A taken along the indicated plane in FIG. 6A. More specifically, FIGS. 6A and 6B depict the lower electrode 414b and the lower insulator 502b of the lower jaw 212. The configuration of the upper jaw 210 (FIGS. 4A and 5A-5B) may be substantially similar, thus the following discussion may be equally applicable to the upper jaw 210. The lower jaw 212 shown in FIGS. 6A-6B may be similar in some respects to the lower jaw 212 shown in FIGS. 5A-5B and therefore may be best understood with reference thereto, where like numerals will represent similar components that may not be described again in detail.

As illustrated, the lower insulator 502b may be overmolded onto the lower electrode 414b, and the lower electrode 414b and the lower insulator 502b cooperatively define the lower slot portion 504b of the knife slot 418. The lower slot portion 504b bifurcates the planar sealing surface 506 of the lower electrode 414b, thereby defining the left and right portions 510a,b of the lower electrode 414b.

As best seen in FIG. 6B, the outer lateral extents 512 of the lower electrode 414b extend away from the planar sealing surface 506 and toward the body of the lower jaw 212 at about a 45° angle, but could alternatively extend at an angle greater or less than 45°, without departing from the scope of the disclosure. The outer lateral extents 512 may also be embedded within the lower insulator 502b, which encapsulates the lateral extents 512 by extending to and terminating at the planar sealing surface 506. As a result, the lower insulator 502b may provide a generally flush interface and finish with the planar sealing surface 506. In the embodiment shown in FIGS. 5A-5B, the lower insulator 502b stops short of the planar sealing surface 506, which leaves the edge 513 (radius, corner, etc.) of the lower electrode 414b exposed. In the illustrated embodiment, however, the edge 513 of the lower electrode 414b is covered by the lower insulator 502b, which finishes flush with the planar sealing surface 506.

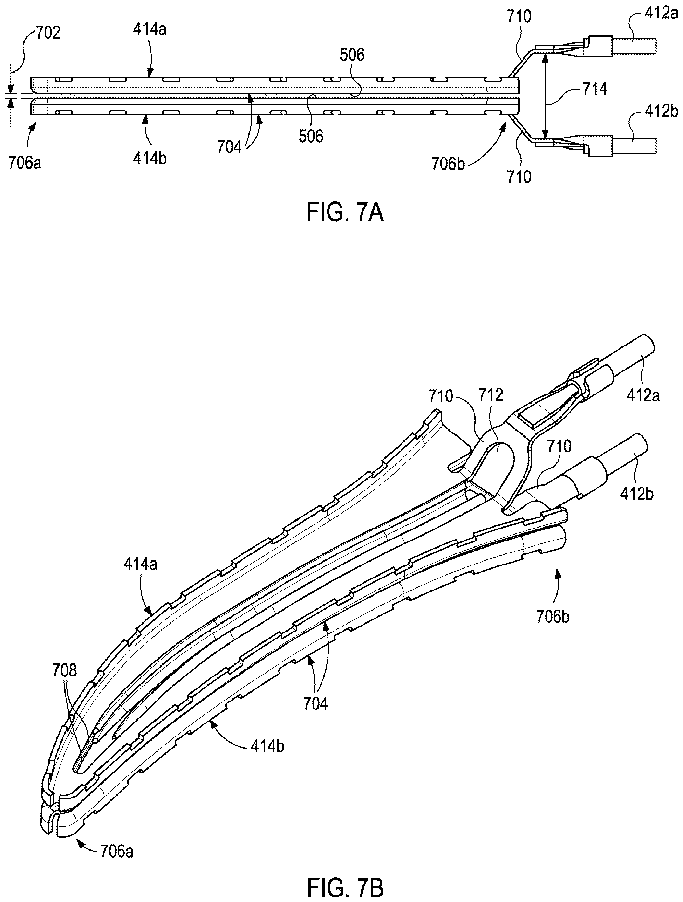

FIGS. 7A and 7B are side and isometric views, respectively, of the upper and lower electrodes 414a,b, according to one or more embodiments. As indicated above, the upper and lower electrodes 414a,b may constitute mirror images of each other. In FIGS. 7A and 7B, the electrodes 414a,b are depicted in a configuration when the jaws 210, 212 (FIGS. 2, 4A, 5A-5B) are in the closed position. In the closed position, the electrodes 414a,b are arranged substantially parallel to each other and a small gap 702 (FIG. 7A) is defined therebetween to receive (accommodate) tissue.

As illustrated, each electrode 414a,b includes an elongate body 704 having a first or "distal" end 706a and a second or "proximal" end 706b opposite the distal end 706a. The body 704 may be made of a variety of rigid, conductive materials, such as a metal. Example conductive materials include, but are not limited to, stainless steel, aluminum, silver, copper, and alloys thereof. Alternatively, stainless steel could also be used as a substrate over which gold, silver, or platinum could be applied through a plating process. An elongate channel 708 (FIG. 7B) is defined in the body 704 of each electrode 414a,b and extends between the distal and proximal ends 706a,b. The elongate channel 708 helps define the knife slot 418 (FIGS. 5A-5B) in the upper and lower jaws 210, 212 (FIGS. 2, 4A, 5A-5B). As best seen in FIG. 7B, the elongate channels 708 substantially align when the jaws 210, 212 are closed.

Each electrode 414a,b may provide an electrical connector 710 positioned at and otherwise extending from the proximal end 706b. The electrical connector 710 provides a location where the first and second electrical conductors 412a,b can be placed in electrical communication with the electrodes 414a,b, respectively. More specifically, the first electrical conductor 412a terminates at and is electrically coupled to the electrical connector 710 of the upper electrode 414a, and the second electrical conductor 412b terminates at and is electrically coupled to the electrical connector 710 of the lower electrode 414b. In contrast to vessel sealers that incorporate a single electrical conductor and relies on a mechanical metallic drive train for the conductive return pathway on an opposing jaw, the presently-described electrical conductors 412a,b are routed directly to the corresponding electrodes 414a,b. While the upper and lower electrodes 414a,b may be mirror images of each other, each electrode 414a,b exhibits a different polarity. As will be appreciated, this direct routing and electrical communication minimizes electrical losses, which enables better signal integrity for driving system response and ensures more controlled impedance values that improve the ability to do distal sensing of tissue properties, and better detection of seal progression.

In some embodiments, the electrical connectors 710 may form an integral part of the corresponding electrode 414a,b from which it extends. In such embodiments, each electrode 414a,b and corresponding electrical connector 710 may comprise a stamped, metal part, which may prove advantageous in simplifying the manufacturing process of the electrodes 414a,b.

Moreover, in at least one embodiment, as illustrated, the elongate channel 708 defined in each electrode 414a,b may extend into the electrical connector 710, which forms or otherwise defines a generally U-shaped passage 712 (FIG. 7B). Consequently, the electrodes 414a,b may be bridged (connected) at both ends 706a,b, which allows for centered, embedded connection of the electrical conductors 412a,b for both electrodes 414a,b.

As best seen in FIG. 7A, the electrical connectors 710 may extend away from the corresponding electrodes 414a,b and, more particularly, away from the plane of the corresponding planar sealing surface 506. In such embodiments, the electrical connectors 710 may extend at about a 45° angle, but could alternatively extend at an angle greater or less than 45°, without departing from the scope of the disclosure. The electrical connectors 710 may extend below the corresponding planar sealing surface 506 such that a gap 714 (FIG. 7A) is defined between the electrical connectors 710. The gap 714 may prove advantageous in providing space to accommodate the knife 416 (FIGS. 4B and 5B) and the knife housing 422 (FIG. 4B). Moreover, the knife 416 may be able to pass through the U-shaped passage 712 provided by each electrical connector 710 as it traverses the knife slot 418 operation.

### Improved Jaw Mechanism

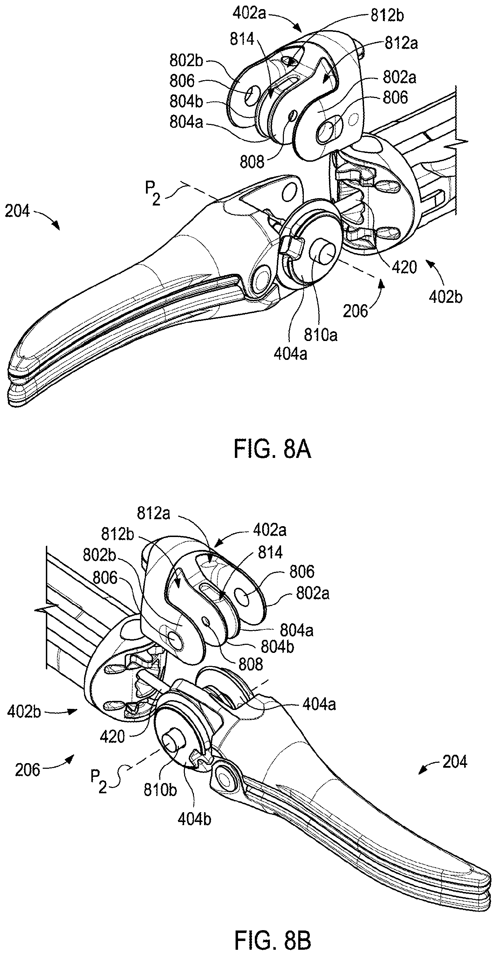

FIGS. 8A and 8B are isometric, partially exploded views of the end effector 204 and the wrist 206 of FIGS. 2 and 4A-4B, as taken from right and left vantage points, respectively. In FIGS. 8A-8B, the distal clevis 402a is shown exploded vertically from the remaining portions of the wrist 206, thus exposing various internal parts of the wrist 206. The drive members 406a-d (FIG. 4A) and the electrical conductors 412a,b (FIGS. 4A-4B) are omitted from FIGS. 8A-8B to enable better viewing of the component parts of the wrist 206.

In the illustrated embodiment, the distal clevis 402a is depicted as a monolithic, one-piece structure, but could alternatively be made of two or more component parts, without departing from the scope of the disclosure. As discussed in more detail below, the proximal end of the distal clevis 402a may be rotatably mounted to the distal end of the proximal clevis 402b, and vice versa, which allows the wrist 206 to articulate in "yaw" about the first pivot axis P₁ (FIG. 4A).

The distal clevis 402a provides and otherwise defines a pair of outer lobes 802a and 802b and a pair of inner lobes 804a and 804b. The outer and inner lobes 802a,b and 804a,b each extend distally, and the inner lobes 804a,b interpose the outer lobes 802a,b. Each outer lobe 802a,b defines an axle aperture 806, and each inner lobe 804a,b defines a pin aperture 808. The axle and pin apertures 806, 808 are co-axially aligned along the second pivot axis P₂ of the wrist 206 and configured to support first and second axles 810a (FIG. 8A) and 810b (FIG. 8B). More specifically, an outer end of the first axle 810a may be configured to be received within the axle aperture 806 of the first outer lobe 802a, and an inner end of the first axle 810a may be configured to be received within the pin aperture 808 of the first inner lobe 804a. Similarly, an outer end of the second axle 810b may be configured to be received within the axle aperture 806 of the second outer lobe 802b, and an inner end of the second axle 810b may be configured to be received within the pin aperture 808 of the second inner lobe 804b.

The first and second pulleys 404a,b may be rotatably mounted to the distal clevis 402a at the first and second axles 810a,b, thereby being able to rotate about the second pivot axis P₂. More specifically, the first pulley 404a may be received within a gap 812a defined between the first outer and inner lobes 802a, 804a and rotatably mounted to the first axle 810a, and the second pulley 404b may be received within a gap 812b defined between the second outer and inner lobes 802b, 804b and configured to be rotatably mounted to the second axle 810b.

In some embodiments, the first and second axles 810a,b may be secured (e.g., welded) to one or both of the axle and pin apertures 806, 808. In such embodiments, the pulleys 404a,b may be rotatably mounted to the first and second axles 810a,b, respectively, such that they are free to rotate. In other embodiments, however, the pulleys 404a,b may be secured (e.g., welded) to the first and second axles 810a,b. In such embodiments, the first and second axles 810a,b may be freely rotatable within the corresponding axle and pin apertures 806, 808. In yet other embodiments, the first and second axles 810a,b may be freely rotatable and not secured to any other component of the wrist 206, without departing from the scope of the disclosure.

As illustrated, a central gap 814 may be defined between the inner lobes 804a,b. The central gap 814 may be configured to accommodate the drive rod 420 and the electrical conductors 412a,b (FIGS. 4A-4B) as extending to the end effector 204.

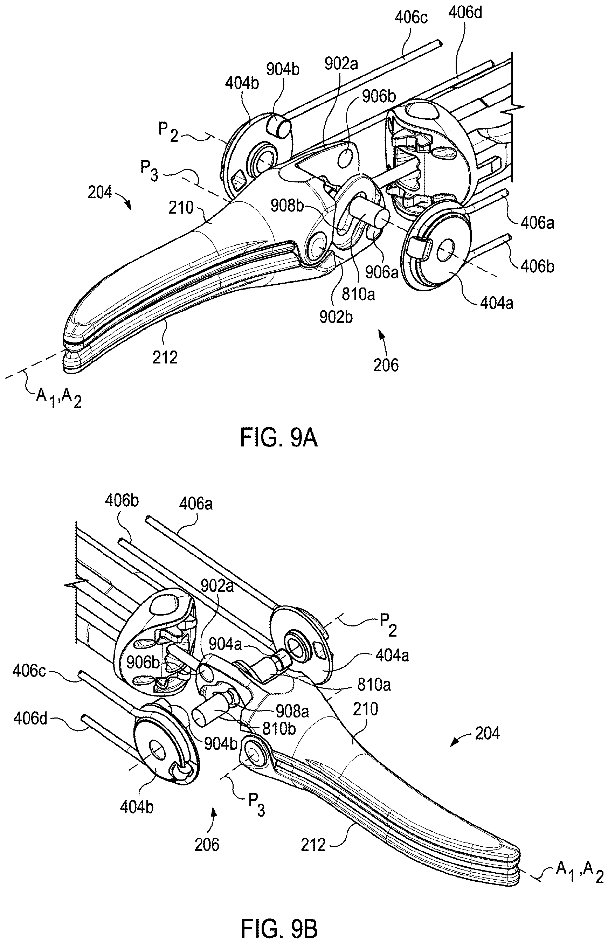

FIGS. 9A and 9B are additional isometric, exploded views of the end effector 204 and the wrist 206 from right and left vantage points, respectively. In FIGS. 9A-9B, the distal clevis 402a (FIGS. 8A-8B) is omitted for simplicity, and the first and second pulleys 404a,b and the drive members 406a-d are shown exploded laterally from the remaining portions of the end effector 204 and the wrist 206. As illustrated, the jaws 210, 212 may be pivotably and rotatably coupled to each other at a third pivot axis P₃, alternately referred to as a "jaw pivot axis". The third pivot axis P₃ may be parallel to the second pivot axis P₂ and orthogonal to the first pivot axis P₁ (FIG. 4A). In at least one embodiment, the longitudinal axes A₁, A₂ of the shaft 202 (FIG. 2) and the end effector 204, respectively, are perpendicular to and intersect the third pivot axis P₃. In some embodiments, as illustrated, both jaws 210, 212 may be configured to simultaneously move and pivot about the jaw pivot axis P₃ to pivot the jaws 210, 212 between the open and closed positions, and may thus be referred to as "bifurcating" jaws. In other embodiments, however, one of the jaws 210, 212 may be configured to pivot or rotate while the other jaw 210, 212 remains stationary, without departing from the scope of the disclosure.

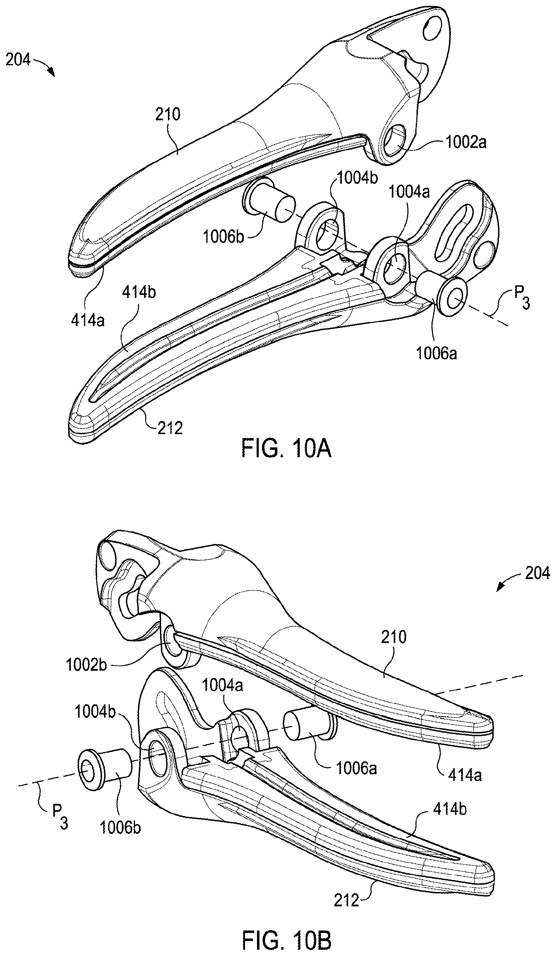

Referring briefly to FIGS. 10A and 10B, illustrated are isometric exploded views of the end effector 204 from left and right vantage points, respectively, according to one or more embodiments. In FIGS. 10A-10B, the upper and lower jaws 210, 212 are shown exploded vertically from each other. As illustrated, the upper jaw 210 provides and otherwise defines first and second jaw axle apertures 1002a and 1002b (FIG. 10B) located at or near the proximal end of the upper jaw 210. Similarly, the lower jaw 212 includes first and second jaw axle apertures 1004a and 1004b located at or near the proximal end of the lower jaw 212.

The jaw axle apertures 1002a,b of the upper jaw 210 are coaxially aligned, and the jaw axle apertures 1004a,b of the lower jaw 212 are also coaxially aligned. When the end effector 204 is properly assembled, the jaw axle apertures 1002a,b of the upper jaw 210 will be aligned coaxially with the jaw axle apertures 1004a,b of the lower jaw 212. More particularly, when the end effector 204 is assembled, the first jaw axle apertures 1002a, 1004a will be juxtaposed against each other, and the second jaw axle apertures 1002b, 1004b will be juxtaposed against each other such that all jaw axle apertures 1002a,b and 1004a,b will be axially aligned along the third pivot axis P₃.

The jaws 210, 212 may be pivotably coupled along the third pivot axis P₃ using one or more jaw axles, shown as a first jaw axle 1006a and a second jaw axle 1006b. The first jaw axle 1006a may be configured to be received within the axially aligned first jaw axle apertures 1002a, 1004a, and the second jaw axle 1006b may be configured to be received within the axially aligned second jaw axle apertures 1002b, 1004b. Once the first and second jaw axles 1006a,b are properly installed, the jaws 210, 212 will be pivotable about the third pivot axis P₃ between the open and closed positions.

Having the jaws 210, 212 rotatably (pivotably) coupled together at the third pivot axis P₃ may prove advantageous for a variety of reasons. First, this can ensure that the center plane, the sealing surfaces (e.g., the planar sealing surfaces 506 of FIGS. 5A-5B), and yaw position of the jaws 210, 212 all remain centered and controlled between the two drive pulleys; e.g., the pulleys 404a,b of FIGS. 8A-8B and 9A-9B. Second, this also reduces components and assembly complexity of the end effector 204, which can result in significant cost savings. Reduction in components will also help to reduce the tolerance stack-ups on jaw gap, which is critical for sealing. And third, this can reduce interfaces for positioning the yaw plane of the end effector 204, thus lowering backlash and reducing sliding friction in closure and yaw motions of the jaws 210, 212.

Moreover, as mentioned above, the longitudinal axes A₁, A₂ (FIGS. 9A-9B) of the shaft 202 (FIG. 2) and the end effector 204, respectively, may perpendicularly intersect (cross through) the third pivot axis P₃. Consequently, the sealing plane of the end effector 204 (e.g., the plane formed by the planar sealing surfaces 506 of FIGS. 5A-5B) may be centered relative to the end effector 204 and the shaft 202 to balance the symmetric jaws 210, 212 that both carry an electrode 414a,b. Having the sealing plane of the end effector 204 at the centerline of the device also allows for equal stiffness of the jaws 210, 212, and thus equal deflection of the jaws 210, 212 under loading.

Referring again to FIGS. 9A-9B, the upper jaw 210 provides a first jaw extension 902a and the lower jaw 212 provides a second jaw extension 902b (FIG. 9A), and each jaw extension 902a,b extends proximally from the corresponding jaws 210, 212. The second pulley 404b may be rotatably coupled (e.g., pinned) to the first jaw extension 902a such that movement (rotation) of the second pulley 404b correspondingly pivots the upper jaw 210 about the third pivot axis P₃. Similarly, the first pulley 404a may be rotatably coupled (e.g. pinned) to the second jaw extension 902b such that movement (rotation) of the first pulley 404a correspondingly pivots the lower jaw 212 about the third pivot axis P₃.

More particularly, in the illustrated embodiment, the first pulley 404a may provide or define a first drive pin 904a (FIG. 9B) configured to mate with a first jaw aperture 906a (FIG. 9A) defined on the second jaw extension 902b, and the second pulley 404b may provide or define a second drive pin 904b configured to mate with a second jaw aperture 906b defined on the first jaw extension 902a. The first and second drive pins 904a,b are eccentric to the second pivot axis P₂ when the pulleys 404a,b are mounted to the jaw axles 810a,b. Consequently, mating the first and second drive pins 904a,b with the second and first jaw apertures 906b,a, respectively, allows the pulleys 404a,b to rotate about the second pivot axis P₂ and simultaneously pivot the jaws 210, 212 about the third pivot axis P₃ and between the open and closed positions.

In an alternative embodiment, the first and second drive pins 904a,b may be provided on the first and second jaw extensions 902a,b, and the first and second jaw apertures 906a,b may be provided on the pulleys 404a,b, or any combination thereof. Moreover, the jaw apertures 906a,b need not be through-holes, as depicted, but could alternatively comprise recesses defined in the jaw extensions 902a,b (or the pulleys 404a,b) and sized and otherwise configured to receive the drive pins 904a,b.

Selective actuation and antagonistic operation of the drive members 406a-d can open or close the jaws 210, 212. More specifically, because the jaws 210, 212 are eccentrically pinned to the pulleys 404a,b, as generally described above, selectively actuating the drive members 406a-d such that the pulleys 404a,b rotate in opposite angular directions may result in the jaws 210, 212 opening or closing about the third pivot axis P₃. Selective actuation and antagonistic operation of the drive members 406a-d may also cause the end effector 204 to articulate at the wrist 206 in both pitch and yaw directions. More particularly, selectively actuating the drive members 406a-d such that the pulleys 404a,b rotate in the same angular direction may result in the jaws 210, 212 pivoting about the second pivot axis P₂ and thereby moving the end effector 204 up or down in pitch. Moreover, selective actuation of a first connected pair of drive members 406a-d while relaxing a second pair of connected drive members 406a-d may cause the end effector 204 to pivot about the first pivot axis P₁ (FIG. 4A) and thereby move in yaw (left or right). The drive pins 904a,b may be optimized to deliver consistent tension in the drive members 406a-d at clamping loads, while maximizing movement.

Still referring to FIGS. 9A-9B, the first jaw extension 902a may further define a first arcuate slot 908a (FIG. 9B), and the second jaw extension 902b may similarly define a second arcuate slot 908b (FIG. 9A). The first axle 810a may be configured to extend through the second arcuate slot 908b, and the second axle 810b may be configured to extend through the first arcuate slot 908a. As the jaws 210, 212 pivot between the closed and open positions, the axles 810a,b traverse the corresponding arcuate slots 908a,b.

In some embodiments, the arcuate slots 908a,b may be used to help prevent over-rotation of the jaws 210, 212 during operation. More specifically, each end of the arcuate slots 908a,b provides and otherwise defines a mechanical hard stop. As the jaws 210, 212 move to the open position, the axles 810a,b will traverse the corresponding arcuate slots 908a,b and may eventually engage the mechanical hard stop at an end of the arcuate slot 908a,b. Engaging the hard stop will prevent the jaws 210, 212 from pivoting further in the open direction, which could result in over-rotation and inadvertently achieving a controls singularity, which could lock the jaws 210, 212. If the drive pins 904a,b are over-rotated to a point that they rotate past (cross over) the longitudinal axis A₁ of the shaft or the longitudinal axis A₂ of the end effector 204 or otherwise become co-axially aligned, this could result in controls singularity, which creates unstable yaw positioning. Reaching controls singularity theoretically provides the jaws 210, 212 with the ability to rotate about different axes, thus eliminating finite control of yaw.

In other embodiments, however, the robotic controllers of the surgical tool 200 (FIG. 2) may be programmed and otherwise configured to prevent over-rotation of the jaws 210, 212 via selective actuation of the drive members 406a,b. In such embodiments, the mechanical hard stops of the arcuate slots 908a,b may serve as a fail-safe mechanism that prevents over-rotation in the event the robotic controllers or programming malfunction.

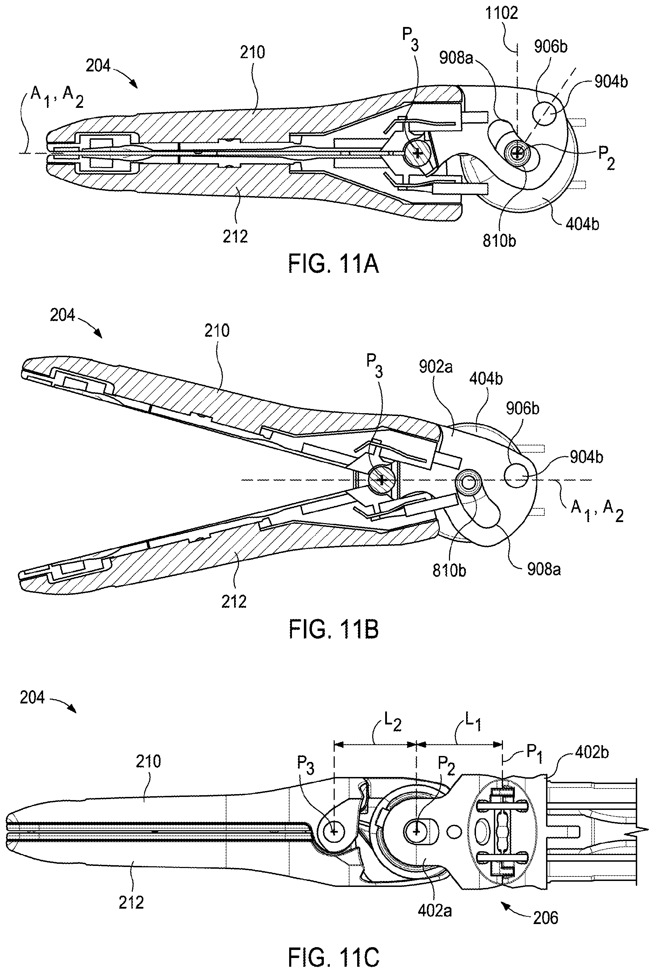

FIGS. 11A-11B are cross-sectional side views of the end effector 204 in the closed and open positions, respectively, according to one or more embodiments. In FIGS. 11A-11B, the second pulley 404b is shown pinned to the first jaw extension 902a of the upper jaw 210, but the following discussion is equally applicable to the first pulley 404a (FIGS. 9A-9B) being pinned to the second jaw extension 902b (FIGS. 9A-9B) of the lower jaw 212. As described above, the second drive pin 904b of the second pulley 404b is configured to mate with the second jaw aperture 906b defined on the first jaw extension 902a such that movement (rotation) of the second pulley 404b correspondingly pivots the upper jaw 210 about the third pivot axis P₃. Moreover, the second axle 810b extends through and traverses the first arcuate slot 908a of the first jaw extension 902a as the jaws 210, 212 pivot between the closed and open positions.

With reference to FIG. 11A, the end effector 204 may be operated in such a manner so as to prevent the second drive pin 904b from rotating "over-center" when approaching or reaching the fully closed position. More particularly, the controls system operating the end effector 204 or the configuration of the first arcuate slot 908a may be configured to prevent the second driver pin 904b from reaching or rotating past a centerline 1102 of the second pulley 404b, where the centerline 1102 is a plane passing through the second pivot axis P₂ and perpendicular to the longitudinal axis A₁ of the shaft 202 (FIG. 2) or the longitudinal axis A₂ of the end effector 204. Approaching the "over-center" condition at the closed position, while not surpassing it, ensures low tension in the drive members 406a-d (FIGS. 9A-9B), while balancing the motion of the drive pin 904b such that jaws 210, 212 do not inadvertently lock in the closed position and instead remain reactive to user input requests for jaw motion. If the second drive pin 904b were somehow able to rotate over-center and past the centerline 1102, the jaws 210, 212 could lock and would require a large amount of force to return from the over-center condition.

In FIG. 11B, the end effector 204 may be operated in such a manner so as to prevent the second drive pin 904b from rotating past (crossing over) a plane extending through the longitudinal axes A₁, A₂, which could result in controls singularity. More particularly, the controls system operating the end effector 204 or the configuration of the first arcuate slot 908a may be configured to prevent the second driver pin 904b from over-rotating past the longitudinal axes A₁, A₂, which could also result in the second drive pin 904b becoming concentrically aligned with the first drive pin 904a (FIGS. 9A-9B) and thereby achieving controls singularity that could lock up the end effector 204 and create unstable yaw positioning.

FIG. 11C is a schematic side view of the end effector 204 and the wrist 206, according to one or more embodiments. As illustrated, and as discussed above, the distal and proximal clevises 402a,b may be rotatably mounted to each other at the first pivot axis P₁ of the wrist 206, which provide "yaw" articulation of the wrist 206, and the jaws 210, 212 are rotatably mounted to the distal clevis 402a at the second or pivot axis P₂, which provides "pitch" articulation of the wrist 206. Moreover, the jaws 210, 212 are pivotably coupled to each other at the third pivot axis P₃, alternately referred to as a "jaw pivot axis".

The first and second pivot axes P₁, P₂ are separated from each other by a first axial length L₁, and the jaw pivot axis P₃ is separated from the second pivot axis P₂ by a second axial length L₂. According to embodiments of the present disclosure, the second axial length L₂ may be equal to or smaller (shorter) than the first axial length L₁. In combination with reduced compliments and simplified linkage of the jaws 210, 210 directly to each other, allows for a shorter mechanism that provides better access and dissection in the articulated postures. Accordingly, having a second axial length L₂ equal to or shorter than the first axial length L₁, may prove advantageous in providing easier access into tight anatomy.

Moreover, in combination with the knife 416 (FIGS. 4B and 5B) and its corresponding knife housing 422 (FIG. 4B) being centrally located within the end effector 204, having the second axial length L₂ equal to or shorter than the first axial length L₁ is an improvement over prior or conventional end effectors. As will be described in greater detail below, the unique architecture and internal structure of the end effector 204 and the distal clevis 402a, and how they are operatively coupled, allows for an open central area that accommodates the knife 416 and the knife housing 422. In contrast, prior end effectors have architecture and internal structure that occludes or extends across any central area, thus eliminating the potential to include a centrally-located knife and knife housing. Attempts to include the knife and knife housing would necessarily result in the second axial length L₂ being longer than the first axial length L₁.

Referring again to FIGS. 9A-9B, with continued reference to FIGS. 11A-11B, in some embodiments the end effector 204 may be designed such that the pulleys 404a,b need only rotate between the centerline 1102 and the longitudinal axes A₁, A₂ (e.g., less than 90°) to obtain about 80-100 Newtons of clamping force.

### Dual Gear Snake Wrist

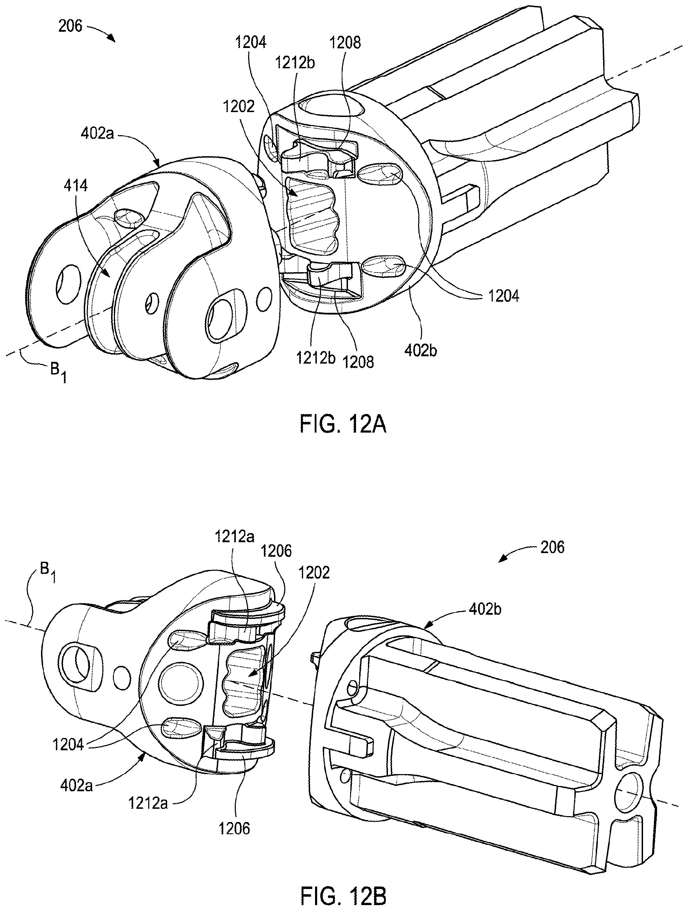

FIGS. 12A and 12B are exploded, isometric views of the distal and proximal clevises 402a,b of the wrist 206, as taken from right and left vantage points, according to one or more embodiments. As mentioned above, the proximal end of the distal clevis 402a may be rotatably mounted to the distal end of the proximal clevis 402b, which allows the wrist 206 to articulate in "yaw" about the first pivot axis P₁ (FIG. 4A). As illustrated, both clevises 402a,b include or define a central passageway 1202 sized to accommodate the electrical conductors 412a,b (FIGS. 4A-4B) and the drive rod 420 (FIGS. 4A-4B). The central passageway 1202 of the distal clevis 402a transitions to the central gap 814 provided on the distal end of the distal clevis 402a. The distal and proximal clevises 402a,b also define a plurality of cable passages 1204 sized to accommodate the drive members 406a-d (FIGS. 4A-4B). When the clevises 402a,b are rotatably coupled and unarticulated, the central passageways 1202 will axially align to enable the electrical conductors 412a,b and the drive rod 420 to extend through the wrist 206, and corresponding cable passages 1204 will axially align to allow the drive members 406a-d to extend through the wrist 206.

In the illustrated embodiment, the distal clevis 402a may provide and otherwise define one or more camming tabs 1206 (two shown in FIG. 12B) configured to be received within and otherwise mated with one or more corresponding camming slots 1208 (shown in FIG. 12A) defined on the proximal clevis 402b. When the clevises 402a,b are rotatably coupled, the camming tabs 1206 will be received within the corresponding camming slots 1208, which may help provide vertical joint alignment. In alternative embodiments, the camming tabs 1206 may be provided on the proximal clevis 402b, and the camming slots 1208 may be provided on the distal clevis 402a, or a combination thereof, without departing from the scope of the disclosure.

In some embodiments, the distal clevis 402a may provide and otherwise define one or more first camming surfaces 1210a (two shown in FIG. 12B), and the proximal clevis 402B may provide and otherwise define one or more second camming surfaces 1210b (two shown in FIG. 12A). The camming surfaces 1210a,b may comprise opposing arcuate or curved surfaces. When the clevises 402a,b are rotatably coupled, the opposing camming surfaces 1210a,b may be in rolling or camming engagement as the wrist 206 articulates. As illustrated, each of the camming surfaces 1210a,b are located above or below the instrument axis B₁.

In the illustrated embodiment, the distal clevis 402a may provide and otherwise define one or more first spur gears 1212a (two shown in FIG. 12B), and the proximal clevis 402b may provide and otherwise define one or more corresponding second spur gears 1212b (two shown in FIG. 12B) configured to interact with the one or more first spur gears 1212a. When the clevises 402a,b are rotatably coupled, the opposing spur gears 1212a,b help the wrist 206 articulate in a controlled manner. As illustrated, the spur gears 1212a,b are each located above or below the instrument axis B₁, which helps improve rotational backlash and better resist torsional loads. Moreover, in at least one embodiment, the cable passages 1204 may be located in the same plane as the spur gears 1221a,b.

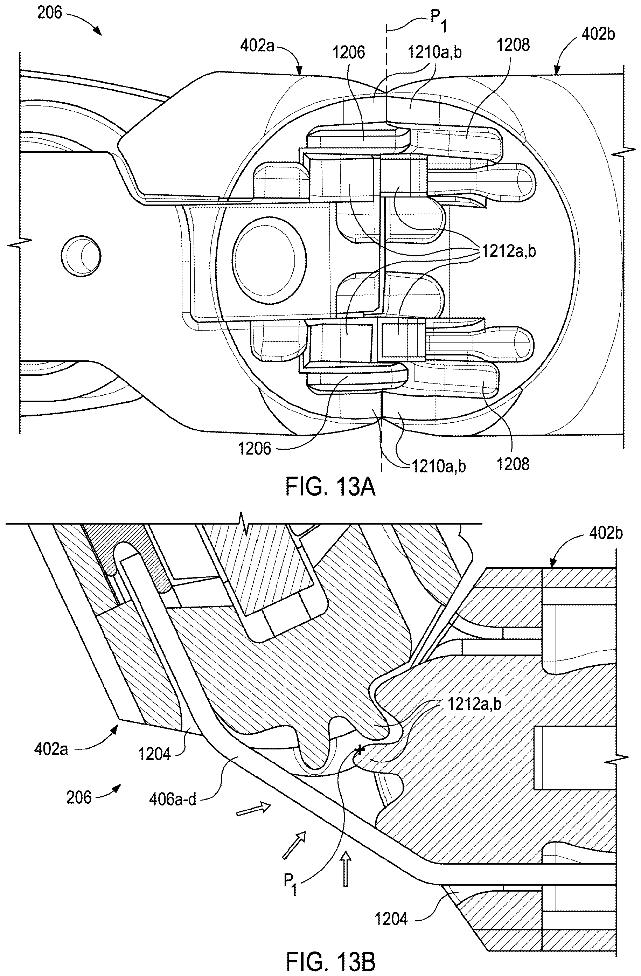

FIG. 13A is an enlarged side view of the wrist 206 in an articulated state, and FIG. 13B is a cross-sectional top view of the wrist 206 in the articulated state, according to one or more embodiments. In FIG. 13A, the distal clevis 402a is rotatably coupled to the proximal clevis 402b, thereby providing the first pivot axis P₁. As illustrated, the camming tabs 1206 provided by the distal clevis 402a are received within and otherwise mated with the corresponding camming slots 1208 provided by the proximal clevis 402b. Moreover, the camming surfaces 1210a,b are in cammed (rolling) engagement with each other as the wrist 206 articulates about the first pivot axis P₁. Furthermore, the spur gears 1212a,b are inter-meshed to help provide the wrist 206 with controlled articulation about the first pivot axis P₁.

In FIG. 13B, the wrist 206 is shown in an articulated state as the distal clevis 402a is rotated relative to the proximal clevis 402b about the first pivot axis P₁. In the illustrated embodiment one of the drive cables 406a-d is shown extended through corresponding cable passages 1204 that allow the drive cable 406a-d to extend through the wrist 206. In at least one embodiment, the drive cable 406a-d may prove advantageous in blocking tissue from becoming entangled in the intermeshed spur gears 1212a,b.

### Knife Housing or "Garage"

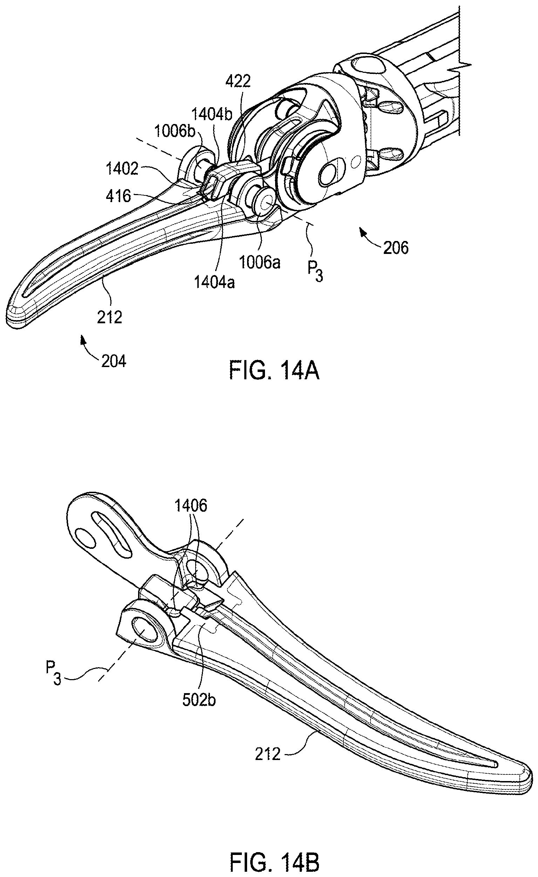

FIG. 14A is another enlarged isometric view of the end effector 204, according to one or more embodiments of the present disclosure. The upper jaw 210 (FIGS. 2, 4A, 8A-8B, and 9A-9B) is omitted from FIG. 14A to enable viewing of various internal features of the end effector 204 and the wrist 206.

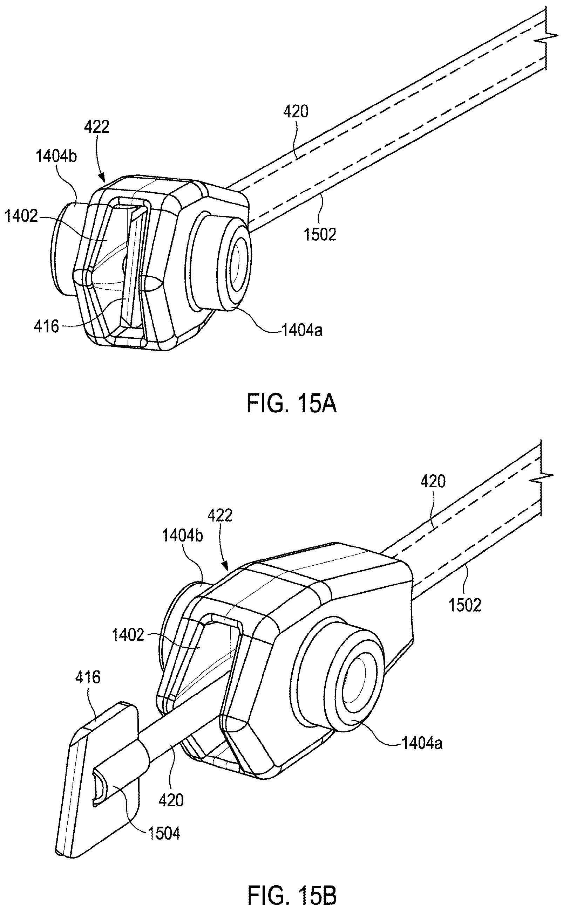

As mentioned above, the knife 416 (mostly occluded) is aligned with and configured to traverse the knife slot 418 defined longitudinally in both the upper and lower jaws 210, 212. The knife housing 422 defines a cavity 1402 sized to receive and "stow" the knife 416 when not in use. The knife 416 is shown in FIG. 14A in a "stowed" position and otherwise received within the cavity 1402. The cavity 1402 provides a conduit through which the drive rod 420 (see FIGS. 15A-15B) is able to extend to move the knife 416 into and along the knife slot 418.

The knife housing 422 may be pivotably mounted to the end effector 204. More particularly, the knife housing 422 may be rotatably mounted between the upper and lower jaws 210, 212 when the jaws 210, 212 are pivotably coupled as described herein with reference to FIGS. 10A-10B. As illustrated, the knife housing 422 may define and otherwise provide opposing first and second bosses 1404a and 1404b (mostly occluded) that laterally protrude from opposing sides of the knife housing 422. When the jaws 210, 212 are pivotably coupled using the first and second jaw axles 1006a,b, as described herein, the bosses 1404a,b will be captured between opposing (e.g., upper and lower) portions of the jaws 210, 212, thus axially securing the knife housing 422 at the end effector 204, but simultaneously allowing the knife housing 422 to pivot relative to the jaws 210, 212 about the third pivot axis P₃.

Accordingly, the knife housing 422 is not fixed (e.g., immovably secured) to any portion of the end effector 204 or the wrist 206, but is instead pivotably secured between opposing central portions of the jaws 210, 212 when the end effector 204 is assembled. Allowing the knife housing 422 to pivot about the third pivot axis P₃ (e.g., due to yaw) may prove advantageous in reducing strain on the knife housing 422 during use.

FIG. 14B is an isometric top view of the lower jaw 212, according to one or more embodiments. For purposes of the present discussion, the upper jaw 210 (FIG. 4A) will include similar features. Consequently, the following discussion of the lower jaw 212 will similarly apply to the upper jaw 210.

As indicated above, the knife housing 422 (FIG. 14A) may be pivotably mounted and captured between opposing portions of the jaws 210, 212. As illustrated, the lower jaw 212 includes and otherwise defines one or more arcuate sections 1406 (two shown). In the illustrated embodiment, the arcuate section(s) 1406 are defined by the lower insulator 502b, but could alternatively be provided by other portions of the lower jaw 212, without departing from the scope of the disclosure. The upper jaw 210 (FIGS. 2, 4A, 8A-8B, and 9A-9B) provides corresponding arcuate section(s) opposing the arcuate sections(s) 1406 when the jaws 210, 212 are pivotably coupled, as described above with reference to FIGS. 10A-10B. Upon pivotably coupling the jaws 210, 212, the opposing first and second bosses 1404a,b (FIG. 14A) of the knife housing 422 (FIG. 14A) will be received between the corresponding arcuate sections 1406 of the upper and lower jaws 210, 212. This will axially secure the knife housing 422 at the end effector 204 (FIG. 14A), while simultaneously allowing the bosses 1404a,b to slidingly engage the opposing arcuate sections 1406 as the end effector 204 is articulated in "pitch" about the third pivot axis P₃.

FIGS. 15A and 15B are enlarged isometric views of the knife 416 and the knife housing 422, according to one or more embodiments. In FIG. 15A, the knife 416 is shown in a first or "stowed" position, where the knife 416 is fully received within the cavity 1402 of the knife housing 422. In FIG. 15B, the knife 416 is shown in a second or "extended" position, where the knife 416 is extended distally out of the cavity 1402. FIGS. 15A-15B also more fully depict the first and second bosses 1404a,b that laterally protrude from opposing sides of the knife housing 422.

As mentioned above, the knife 416 may be operatively coupled to the distal end of the drive rod 420 (shown in dashed lines in FIG. 15A). In some embodiments, the drive rod 420 may comprise a solid shaft, but may alternatively comprise a tube or tubular structure. Moreover, the drive rod 420 may be made of a variety of flexible materials including, but not limited to, a metal or metal alloy (e.g., a nickel-titanium alloy or "nitinol"), a plastic or thermoplastic material, a composite material, or any combination thereof. The drive rod 420 may also comprise a braided cable construction of a metal (e.g., stainless steel), or any of the aforementioned materials, and such braided cable may be designed and radially constrained to support axial loads.

In some embodiments, as illustrated, a flexible sheath 1502 (e.g., a hypotube or the like) may cover all or a portion of the drive rod 420. The sheath 1502 may support and help prevent buckling of the drive rod 420 upon assuming compressive loads during articulation of the wrist 206 and opening and closure of the jaws 210, 212. Similar to the drive rod 420, the flexible sheath 1502 may be made of a variety of flexible materials including, but not limited to, a metal or metal alloy (e.g., a nickel-titanium alloy or "nitinol"), a metallic coil, a plastic or thermoplastic material, a composite material, or any combination thereof.

Upon firing the end effector 204 (FIG. 14), the drive rod 420 is moved (urged) distally, which correspondingly moves the knife 416 to the extended position and otherwise out of the cavity 1402 and into the knife slot 418 (FIG. 14). As the drive rod 420 translates distally, the sheath 1502 supports the drive rod 420 against axial buckling resulting from compressive loading on the drive rod 420. After firing is complete, the drive rod 420 is retracted proximally, which correspondingly pulls the knife 416 proximally and back to the stowed position and otherwise into the cavity 1402 until it is desired to again fire the end effector 204.

As best seen in FIG. 15B, the knife 416 may be attached to the distal end of the drive rod 420 at a retention feature 1504. The retention feature 1504 may comprise any attachment or coupling means that removably or permanently fixes the knife 416 to the drive rod 420. For example, the retention feature 1504 may comprise, but is not limited to, a welded interface, an adhesive attachment, an interference or shrink fit, an overmold (e.g., a shaped block of material or a support block), one or more mechanical fasteners, or any combination thereof. In at least one embodiment, the retention feature 1504 may comprise a formed shape on the drive rod 420 or alternatively on the knife 416.

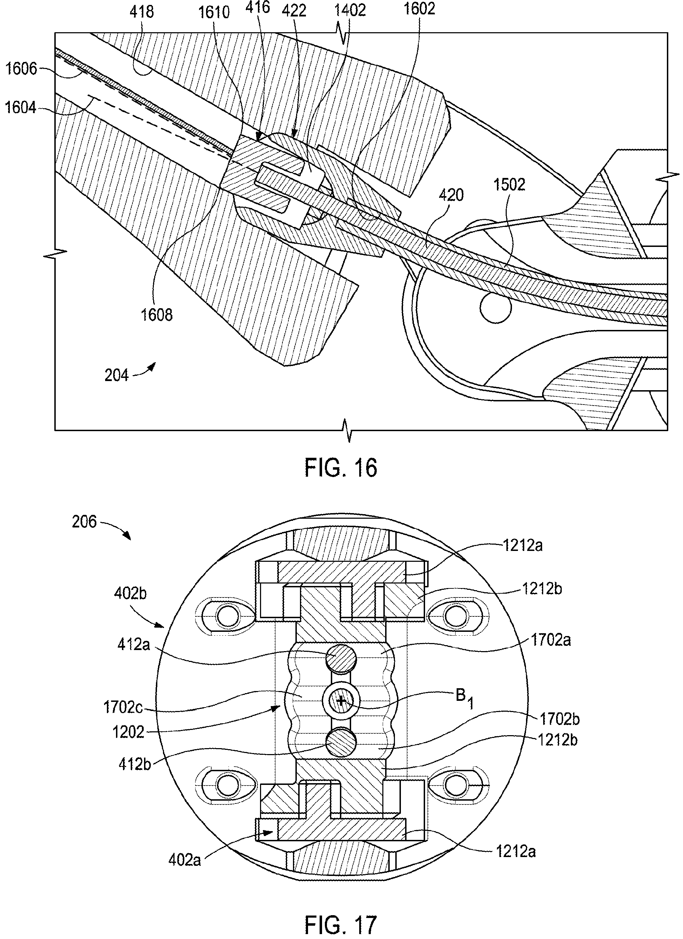

FIG. 16 is an enlarged cross-sectional side view of portions of the end effector 204 and the wrist 206, according to one or more embodiments. As illustrated, the knife 416 is at least partially received within the cavity 1402 of the knife housing 422, and the drive rod 420 is at least partially received within the flexible sheath 1502.

The knife housing 422 may define a central conduit 1602 that extends to and communicates with the cavity 1402. The drive rod 420 is extendable through the central conduit 1602 and able to translate longitudinally therethrough when transitioning the knife 416 between the stowed and extended positions. In some embodiments, the distal end of the sheath 1502 may be received within the central conduit 1602. In at least one embodiment, for example, the knife housing 422 may be secured to the distal end of the sheath 1502, such as being overmolded onto the sheath 1502. In other embodiments, the knife housing 422 may be secured to the distal end of the sheath 1502 via welding, an adhesive, a shrink-fit or interference engagement, or any combination of the foregoing.

In FIG. 16, the end effector 204 is articulated in pitch and the knife 416 is in the process of transitioning from the stowed position to the extended position. Articulating the end effector 204 also causes the knife housing 422 to pivot about the third pivot axis P₃ (FIG. 14), which results in a line of action 1604 (dashed lines) of the drive rod 422 becoming non-parallel to a line of action 1606 (dashed lines) of the knife 416 when the knife 416 exits the knife housing 422 and transitions into the knife slot 418.

In some embodiments, to help ease the transition from the cavity 1402 to the knife slot 418 when the end effector 204 is articulated, at least one of the leading corners 1608, 1610 of the knife 416 may be rounded or chamfered. In the illustrated embodiment, the lower leading corner 1608 of the knife 416 is rounded, while the upper leading corner 1610 defines a sharp angle. As will be appreciated, the rounded, lower leading corner 1608 may prove advantageous in creating a smooth sliding transition for the knife 416 as it enters the knife track 418.

In other embodiments, the lower leading corner 1608 may define a sharp angle, and the upper leading corner 1610 may alternatively be rounded or chamfered. In yet other embodiments, both leading corners 1608, 1610 may be rounded or chamfered, without departing from the scope of the disclosure.

As illustrated, the drive rod 420 is directly pushed without any redirect pulleys being used in the end effector 204. Those skilled in the art will readily appreciate that this can simplify the proximal end of the wrist 206 and the instrument as a whole, which can reduce costs.

FIG. 17 is a cross-sectional end view of the wrist 206 as taken through the first pivot axis P₁ (FIG. 4A), according to one or more embodiments. As discussed above, the proximal end of the distal clevis 402a is rotatably mounted to the distal end of the proximal clevis 402b at the first pivot axis P₁, which allows the wrist 206 to articulate in "yaw" about the first pivot axis P₁. A cross-section of the first spur gears 1212a of the distal clevis 402a are shown intermeshed with a cross-section of the second spur gears 1212b of the proximal clevis 402b, as generally described above.

Also shown in FIG. 17 is the central passageway 1202 of the proximal clevis 402b, which is sized to accommodate the electrical conductors 412a,b and the drive rod 420. While not shown, a similar central passageway may be defined in the distal clevis 402a and may axially align with the central passageway 1202 of the proximal clevis 402b. In some embodiments, as illustrated, the central passageway 1202 may flare open and define one or more arcuate transition surfaces, shown as a first arcuate transition surface 1702a, a second arcuate transition surface 1702b, and a third arcuate transition surface 1702c. The arcuate transition surfaces 1702a-c may be configured to receive and engage the electrical conductors 412a,b and the drive rod 420 as the wrist 206 articulates in "yaw" about the first pivot axis P₁. The arcuate transition surfaces 1702a-c may prove advantageous in providing a smooth and curved transition for the electrical conductors 412a,b and the drive rod 420 as the wrist 206 articulates in "yaw," as opposed to sharp transition surfaces, which could fatigue the material of the electrical conductors 412a,b and the drive rod 420 over time.

As illustrated, the drive rod 420 extends along the instrument axis B₁, and the electrical conductors 412a,b are arranged on radially opposite sides (e.g., above and below) of the drive rod 420. In the illustrated embodiment, the electrical conductors 412a,b are arranged above and below the drive rod 420, but could alternatively be arranged on opposing left and right sides of the drive rod 420, without departing from the scope of the disclosure. Accordingly, the drive rod 420 may be centered (centrally-located) relative to the cross-section of the wrist 206, which can help ensure that the controls length compensation is symmetric and the parasitic load imparted on the articulation system is symmetric, such that clamp force variation relative to a particular pose of the end effector 204 is consistent and predictable.

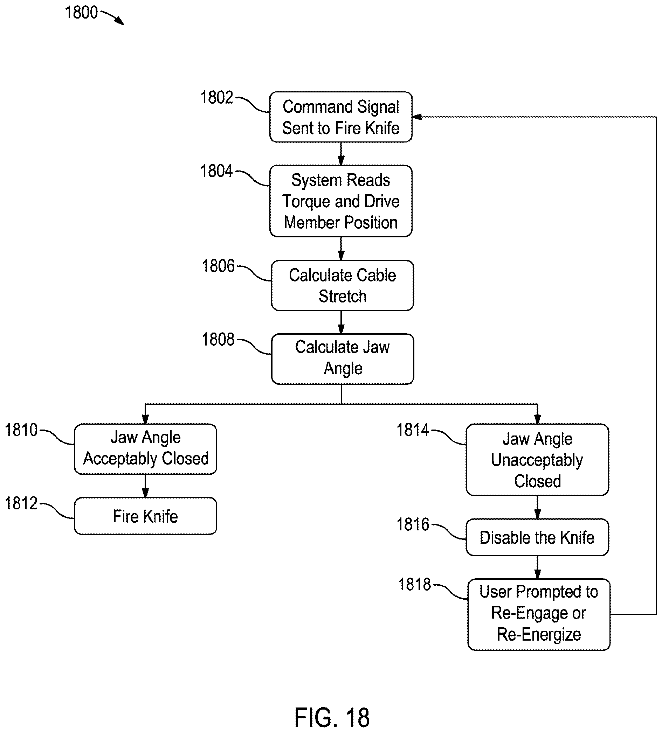

FIG. 18 is a schematic flowchart of an example process algorithm 1800 in accordance with one or more embodiments of the present disclosure. The process algorithm 1800 may be used to infer jaw position based on closure cable position and load, which can ensure that the tips of the jaws 210, 212 are fully approximated and at an angle safe for firing. As will be appreciated, this can help prevent the knife 416 from escaping the knife slot 418 in an over-stuffed jaw condition.

As shown in the process algorithm 1800, a user sends a command signal to request firing of the knife 416, as at 1802. The user may send the command signal to the robotic surgical system 100 (FIG. 1), for example, and more particularly, to the control computer 104 (FIG. 1). The system will read the torque and the position of the drive members in preparation for closure of the jaws 210, 212, as at 1804. Based on the measured torque, the system will calculate cable stretch, as at 1806, and based on the calculated cable length, and the actuator position relative to home, the position of the jaws 210, 212 (the real aperture angle between the two jaws 210, 212) may then be calculated by the system, as at 1808. If the jaw angle between the jaws 210, 212 is determined to be acceptably closed, as at 1810, then the system will proceed to fire the knife 416, as at 1812.

If, however, the jaw angle between the jaws 210, 212 is determined to be un-acceptably closed, as at 1814, the system may be programmed to disable the knife 416, as at 1816. As will be appreciated, if the knife 416 were to be fired (extended along the knife slot 418) with the jaws 210, 212 open past a predetermined angle, the knife 416 could be completely exposed and potentially dislodge from the knife slot 418. Upon disablement of the knife 416, the user may then be prompted to reengage the tissue between the jaws 210, 212 or re-energize the electrodes 414a,b, as at 1818. The process algorithm 1800 may then return to the first step, as at 1802, and the process will repeat itself.

Embodiments disclosed herein include:
A. An end effector that includes a first jaw having a first electrode and a first insulator secured thereto, a second jaw having a second electrode and a second insulator secured thereto, the first and second jaws being pivotable between open and closed positions, and a knife slot cooperatively defined in the first and second jaws and defining a diamond-shape cross-section when the first and second jaws are in the closed position, wherein each electrode provides an inner lateral extent that cooperatively defines the diamond-shape cross-section, and wherein a length of at least one of the inner lateral extents is greater than a thickness of a corresponding one of the first or second electrodes.
B. A surgical tool includes a drive housing, an elongate shaft that extends from the drive housing, and an end effector arranged at a distal end of the elongate shaft. The end effector includes a first jaw having a first electrode and a first insulator secured thereto, a second jaw having a second electrode and a second insulator secured thereto, the first and second jaws being pivotable between open and closed positions, and a knife slot cooperatively defined in the first and second jaws and defining a diamond-shape cross-section when the first and second jaws are in the closed position. The surgical tool further includes a drive rod extending from the drive housing and terminating at the end effector, and a knife operatively coupled to the drive rod and extendable into the knife slot, wherein the drive rod is actuatable to longitudinally move the knife within the knife slot, wherein each electrode provides an inner lateral extent that cooperatively defines the diamond-shape cross-section when the first and second jaws are in the closed position, and wherein a length of at least one of the inner lateral extents is greater than a thickness of a corresponding one of the first or second electrodes.

Each of embodiments A and B may have one or more of the following additional elements in any combination: Element 1: wherein the first insulator and the first electrode cooperatively define a first slot portion that bifurcates a first planar surface of the first electrode into first left and right portions, and the second insulator and the second electrode cooperatively define a second slot portion that bifurcates a second planar surface of the second electrode into second left and right portions, and wherein the first and second slot portions cooperatively define the knife slot when the first and second jaws are in the closed position. Element 2: wherein the first left and right portions each provide an outer lateral extent extending away from the first planar surface and embedded within the first insulator, and the second left and right portions each provide an outer lateral extent extending away from the second planar surface and embedded within the second insulator. Element 3: wherein each outer lateral extent provides an electrically exposed edge that provides a transition between the outer lateral extent and the first and second planar sealing surfaces. Element 4: wherein the outer lateral extents are embedded within the first and second insulators such that the first and second insulators provide a flush interface with the first and second planar sealing surfaces, respectively. Element 5: wherein the first left and right portions each provide the inner lateral extent extending away from the first planar surface, and the second left and right portions each provide the inner lateral extent extending away from the second planar surface. Element 6: wherein the inner lateral extents of the first and second left and right portions cooperatively define the diamond-shape cross-section when the first and second jaws are in the closed position. Element 7: wherein each of the insulators defines a trough section extending laterally across the knife slot and thereby structurally connecting lateral sides of the first and second insulators. Element 8: wherein each electrode provides an elongate body with opposing distal and proximal ends, and an elongate channel is defined in the body and extends between the distal and proximal ends to form part of the knife slot. Element 9: further comprising a first electrical connector extending from the proximal end of the first electrode and configured to receive a first electrical conductor to provide electrical energy to the first electrode, and a second electrical connector extending from the proximal end of the second electrode and configured to receive a second electrical conductor to provide electrical energy to the second electrode. Element 10: wherein each electrical connector defines a generally U-shaped passage such that each electrode is connected at both the distal and proximal ends, and wherein the knife is extendable into the knife slot by extending at least partially through the U-shaped passage of each electrical connector. Element 11: wherein the first and second electrical connectors extend away from corresponding planar sealing surfaces of the first and second electrodes, respectively, such that a gap is defined between the first and second electrical connectors that accommodates the knife.

Element 12: wherein the first insulator and the first electrode cooperatively define a first slot portion that bifurcates a first planar surface of the first electrode, and the second insulator and the second electrode cooperatively define a second slot portion that bifurcates a second planar surface of the second electrode, and wherein the first and second slot portions cooperatively define the knife slot when the first and second jaws are in the closed position. Element 13: wherein the first planar surface provides opposing outer lateral extents extending away from the first planar surface and embedded within the first insulator, and the second planar surface provides opposing outer lateral extents extending away from the second planar surface and embedded within the second insulator. Element 14: wherein the inner lateral extent of each electrode is provided by the first and second planar surfaces and each inner lateral extent extends away from the first and second planar surfaces, respectively. Element 15: wherein each electrode provides an elongate body with opposing distal and proximal ends, and an elongate channel is defined in the body and extends between the distal and proximal ends to form part of the knife slot. Element 16: further comprising a first electrical connector extending from the proximal end of the first electrode, a first electrical conductor extending from the drive housing and coupled to the first electrical connector to provide electrical energy to the first electrode, a second electrical connector extending from the proximal end of the second electrode, and a second electrical conductor extending from the drive housing and coupled to the second electrical connector to provide electrical energy to the second electrode. Element 17: wherein each electrical connector defines a generally U-shaped passage such that each electrode is connected at both the distal and proximal ends, and wherein the knife is extendable into the knife slot by extending at least partially through the U-shaped passage of each electrical connector. Element 19: wherein the first and second electrical connectors extend away from corresponding planar sealing surfaces of the first and second electrodes, respectively, such that a gap is defined between the first and second electrical connectors that accommodates the knife.

By way of non-limiting example, exemplary combinations applicable to A and B include: Element 1 with Element 2; Element 2 with Element 3; Element 2 with Element 4; Element 1 with Element 5; Element 5 with Element 6; Element 8 with Element 9; Element 9 with Element 10; Element 9 with Element 11; Element 12 with Element 13; Element 12 with Element 14; Element 15 with Element 16; Element 16 with Element 17; and Element 16 with Element 18.

Therefore, the disclosed systems and methods are well adapted to attain the ends and advantages mentioned as well as those that are inherent therein. The particular embodiments disclosed above are illustrative only, as the teachings of the present disclosure may be modified and practiced in different but equivalent manners apparent to those skilled in the art having the benefit of the teachings herein. Furthermore, no limitations are intended to the details of construction or design herein shown, other than as described in the claims below. It is therefore evident that the particular illustrative embodiments disclosed above may be altered, combined, or modified and all such variations are considered within the scope of the present disclosure. The systems and methods illustratively disclosed herein may suitably be practiced in the absence of any element that is not specifically disclosed herein and/or any optional element disclosed herein. While compositions and methods are described in terms of "comprising," "containing," or "including" various components or steps, the compositions and methods can also "consist essentially of" or "consist of" the various components and steps. All numbers and ranges disclosed above may vary by some amount. Whenever a numerical range with a lower limit and an upper limit is disclosed, any number and any included range falling within the range is specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an," as used in the claims, are defined herein to mean one or more than one of the elements that it introduces.

As used herein, the phrase "at least one of" preceding a series of items, with the terms "and" or "or" to separate any of the items, modifies the list as a whole, rather than each member of the list (i.e., each item). The phrase "at least one of" allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

## Claims

1. An end effector, comprising:
a first jaw (210) having a first electrode (414a) and a first insulator (502a) secured thereto;
a second jaw (212) having a second electrode (414b) and a second insulator (502b) secured thereto, the first and second jaws (210, 212) being pivotable between open and closed positions; and
a knife slot (418) cooperatively defined in the first and second jaws (210, 212) and defining a diamond-shape cross-section when the first and second jaws are in the closed position,
wherein each electrode provides an inner lateral extent (514) that cooperatively defines the diamond-shape cross-section, and
wherein a length of at least one of the inner lateral extents (514) is greater than a thickness of a corresponding one of the first or second electrodes (414a, 414b).

2. The end effector of claim 1, wherein the first insulator (502a) and the first electrode (414a) cooperatively define a first slot portion (504a) that bifurcates a first planar surface of the first electrode into first left and right portions (508a, 508b), and the second insulator and the second electrode cooperatively define a second slot portion (504b) that bifurcates a second planar surface of the second electrode into second left and right portions (510a, 510b), and
wherein the first and second slot portions (504a, 504b) cooperatively define the knife slot when the first and second jaws are in the closed position.

3. The end effector of claim 2, wherein the first left and right portions (508a, 508b) each provide an outer lateral extent (512) extending away from the first planar surface and embedded within the first insulator, and the second left and right portions each provide an outer lateral extent extending away from the second planar surface and embedded within the second insulator, optionally wherein each outer lateral extent provides an electrically exposed edge that provides a transition between the outer lateral extent and the first and second planar sealing surfaces.

4. The end effector of claim 3, wherein the outer lateral extents (512) are embedded within the first and second insulators such that the first and second insulators provide a flush interface with the first and second planar sealing surfaces, respectively.

5. The end effector of any one of claims 2 to 4, wherein the first left and right portions (508a, 508b) each provide the inner lateral extent extending away from the first planar surface, and the second left and right portions (510a, 510b) each provide the inner lateral extent extending away from the second planar surface, optionally wherein the inner lateral extents of the first and second left and right portions cooperatively define the diamond-shape cross-section when the first and second jaws are in the closed position.

6. The end effector of any preceding claim, wherein each of the insulators defines a trough section (516) extending laterally across the knife slot and thereby structurally connecting lateral sides of the first and second insulators.

7. The end effector of any preceding claim, wherein each electrode (414a, 414b) provides an elongate body with opposing distal and proximal ends, and an elongate channel is defined in the body and extends between the distal and proximal ends to form part of the knife slot.

8. The end effector of claim 7, further comprising:
a first electrical connector (710) extending from the proximal end of the first electrode and configured to receive a first electrical conductor to provide electrical energy to the first electrode; and
a second electrical connector extending from the proximal end of the second electrode and configured to receive a second electrical conductor to provide electrical energy to the second electrode.

9. A surgical tool, comprising:
a drive housing (208);
an elongate shaft (202) that extends from the drive housing;
the end effector of claim 1;
a drive rod (420) extending from the drive housing and terminating at the end effector; and
a knife (416) operatively coupled to the drive rod and extendable into the knife slot, wherein the drive rod is actuatable to longitudinally move the knife within the knife slot.

10. The surgical tool of claim 9, wherein the first insulator and the first electrode (414a) cooperatively define a first slot portion (504a) that bifurcates a first planar surface of the first electrode, and the second insulator and the second electrode cooperatively define a second slot portion (504b) that bifurcates a second planar surface of the second electrode, and
wherein the first and second slot portions cooperatively define the knife slot when the first and second jaws are in the closed position, optionally wherein the first planar surface provides opposing outer lateral extents extending away from the first planar surface and embedded within the first insulator, and the second planar surface provides opposing outer lateral extents extending away from the second planar surface and embedded within the second insulator.

11. The surgical tool of claim 10, wherein the inner lateral extent of each electrode is provided by the first and second planar surfaces and each inner lateral extent extends away from the first and second planar surfaces, respectively.

12. The surgical tool of any one of claims 9 to 11, wherein each electrode provides an elongate body with opposing distal and proximal ends, and an elongate channel is defined in the body and extends between the distal and proximal ends to form part of the knife slot (418).

13. The surgical tool of claim 12, further comprising:
a first electrical connector extending from the proximal end of the first electrode;
a first electrical conductor extending from the drive housing and coupled to the first electrical connector to provide electrical energy to the first electrode;
a second electrical connector extending from the proximal end of the second electrode; and
a second electrical conductor extending from the drive housing and coupled to the second electrical connector to provide electrical energy to the second electrode.

14. The end effector of claim 8 or the surgical tool of claim 13, wherein each electrical connector defines a generally U-shaped passage such that each electrode is connected at both the distal and proximal ends, and wherein the knife is extendable into the knife slot (418) by extending at least partially through the U-shaped passage of each electrical connector.

15. The end effector of claim 8 or the surgical tool of claim 13 or claim 14, wherein the first and second electrical connectors extend away from corresponding planar sealing surfaces of the first and second electrodes (414a, 414b), respectively, such that a gap is defined between the first and second electrical connectors that accommodates the knife.

## Patentansprüche

1. Endeffektor, umfassend:
eine erste Backe (210), die eine erste Elektrode (414a) und einen ersten Isolator (502a) aufweist, der daran befestigt ist;
eine zweite Backe (212), die eine zweite Elektrode (414b) und einen zweiten Isolator (502b) aufweist, der daran befestigt ist, wobei die erste und die zweite Backe (210, 212) zwischen einer offenen und einer geschlossenen Position schwenkbar sind; und
einen Messerschlitz (418), der in der ersten und der zweiten Backe (210, 212) zusammenwirkend definiert ist und einen rautenförmigen Querschnitt definiert, wenn die erste und die zweite Backe in der geschlossenen Position sind,
wobei jede Elektrode eine innere seitliche Erstreckung (514) aufweist, die den rautenförmigen Querschnitt zusammenwirkend definiert, und
wobei eine Länge von mindestens einer der inneren seitlichen Erstreckungen (514) größer als eine Dicke einer entsprechenden einen von der ersten oder der zweiten Elektrode (414a, 414b) ist.

2. Endeffektor nach Anspruch 1, wobei der erste Isolator (502a) und die erste Elektrode (414a) einen ersten Schlitzabschnitt (504a) zusammenwirkend definieren, der eine erste ebene Oberfläche der ersten Elektrode in einen ersten linken und rechten Abschnitt (508a, 508b) gabelt, und der zweite Isolator und die zweite Elektrode einen zweiten Schlitzabschnitt (504b) zusammenwirkend definieren, der eine zweite ebene Oberfläche der zweiten Elektrode in einen zweiten linken und rechten Abschnitt (510a, 510b) gabelt, und
wobei der erste und der zweite Schlitzabschnitt (504a, 504b) den Messerschlitz zusammenwirkend definieren, wenn sich die erste und die zweite Backe in der geschlossenen Position befinden.

3. Endeffektor nach Anspruch 2, wobei der erste linke und rechte Abschnitt (508a, 508b) jeweils eine äußere seitliche Erstreckung (512) aufweisen, die sich von der ersten ebenen Oberfläche weg erstreckt und innerhalb des ersten Isolators eingebettet ist, und der zweite linke und rechte Abschnitt jeweils eine äußere seitliche Erstreckung aufweisen, die sich von der zweiten ebenen Oberfläche weg erstreckt und innerhalb des zweiten Isolators eingebettet ist, optional wobei jede äußere seitliche Erstreckung eine elektrisch freiliegende Kante bereitstellt, die einen Übergang zwischen der äußeren seitlichen Erstreckung und der ersten und der zweiten ebenen Dichtungsoberfläche bereitstellt.

4. Endeffektor nach Anspruch 3, wobei die äußeren seitlichen Erstreckungen (512) innerhalb des ersten und des zweiten Isolators derart eingebettet sind, dass der erste und der zweite Isolator eine bündige Schnittstelle mit der ersten beziehungsweise zweiten ebenen Dichtungsoberfläche bereitstellen.

5. Endeffektor nach einem der Ansprüche 2 bis 4, wobei der erste linke und rechte Abschnitt (508a, 508b) jeweils die innere seitliche Erstreckung bereitstellen, die sich von der ersten ebenen Oberfläche weg erstreckt, und der zweite linke und rechte Abschnitt (510a, 510b) jeweils die innere seitliche Erstreckung bereitstellen, die sich von der zweiten ebenen Oberfläche weg erstreckt, optional wobei die inneren seitlichen Erstreckungen des ersten und des zweiten linken und rechten Abschnitts den rautenförmigen Querschnitt zusammenwirkend definieren, wenn sich die erste und die zweite Backe in der geschlossenen Position befinden.

6. Endeffektor nach einem der vorstehenden Ansprüche, wobei jeder der Isolatoren einen Trogbereich (516) definiert, der sich seitlich über den Messerschlitz hinweg erstreckt und wobei dadurch die seitlichen Seiten des ersten und des zweiten Isolators strukturell angeschlossen werden.

7. Endeffektor nach einem der vorstehenden Ansprüche, wobei jede Elektrode (414a, 414b) einen länglichen Körper mit einem gegenüberliegenden distalen und proximalen Ende bereitstellt und ein länglicher Kanal in dem Körper definiert ist und sich zwischen dem distalen und dem proximalen Ende erstreckt, um einen Teil des Messerschlitzes auszubilden.

8. Endeffektor nach Anspruch 7, ferner umfassend:
einen ersten elektrischen Anschluss (710), der sich von dem proximalen Ende der ersten Elektrode erstreckt und konfiguriert ist, um einen ersten elektrischen Leiter aufzunehmen, um der ersten Elektrode elektrische Energie bereitzustellen; und
einen zweiten elektrischen Anschluss, der sich von dem proximalen Ende der zweiten Elektrode erstreckt und konfiguriert ist, um einen zweiten elektrischen Leiter aufzunehmen, um der zweiten Elektrode elektrische Energie bereitzustellen.

9. Chirurgisches Werkzeug, umfassend:
ein Antriebsgehäuse (208);
einen länglichen Schaft (202), der sich von dem Antriebsgehäuse erstreckt;
der Endeffektor nach Anspruch 1;
eine Antriebsstange (420), die sich von dem Antriebsgehäuse erstreckt und an dem Endeffektor endet; und
ein Messer (416), das mit der Antriebsstange wirkgekoppelt ist und in den Messerschlitz erstreckbar ist, wobei die Antriebsstange betätigbar ist, um das Messer in Längsrichtung innerhalb des Messerschlitzes zu bewegen.

10. Chirurgisches Werkzeug nach Anspruch 9, wobei der erste Isolator und die erste Elektrode (414a) einen ersten Schlitzabschnitt (504a) zusammenwirkend definieren, der eine erste ebene Oberfläche der ersten Elektrode gabelt, und der zweite Isolator und die zweite Elektrode einen zweiten Schlitzabschnitt (504b) zusammenwirkend definieren, der eine zweite ebene Oberfläche der zweiten Elektrode gabelt, und
wobei der erste und der zweite Schlitzabschnitt den Messerschlitz zusammenwirkend definieren, wenn sich die erste und die zweite Backe in der geschlossenen Position befinden, optional wobei die erste ebene Oberfläche gegenüberliegende äußere seitliche Erstreckungen bereitstellt, die sich von der ersten ebenen Oberfläche weg erstrecken und innerhalb des ersten Isolators eingebettet sind, und die zweite ebene Oberfläche gegenüberliegende äußere seitliche Erstreckungen aufweist, die sich von der zweiten ebenen Oberfläche weg erstrecken und innerhalb des zweiten Isolators eingebettet sind.

11. Chirurgisches Werkzeug nach Anspruch 10, wobei die innere seitliche Erstreckung jeder Elektrode durch die erste und die zweite ebene Oberfläche bereitgestellt wird und jede innere seitliche Erstreckung sich jeweils von der ersten beziehungsweise der zweiten ebenen Oberfläche weg erstreckt.

12. Chirurgisches Werkzeug nach einem der Ansprüche 9 bis 11, wobei jede Elektrode einen länglichen Körper mit dem gegenüberliegenden distalen und proximalen Ende bereitstellt und ein länglicher Kanal in dem Körper definiert ist und sich zwischen dem distalen und dem proximalen Ende erstreckt, um einen Teil des Messerschlitzes (418) auszubilden.

13. Chirurgisches Instrument nach Anspruch 12, ferner umfassend:
einen ersten elektrischen Anschluss, der sich von dem proximalen Ende der ersten Elektrode erstreckt;
einen ersten elektrischen Leiter, der sich von dem Antriebsgehäuse erstreckt und mit dem ersten elektrischen Anschluss gekoppelt ist, um der ersten Elektrode elektrische Energie bereitzustellen;
einen zweiten elektrischen Anschluss, der sich von dem proximalen Ende der zweiten Elektrode erstreckt; und
einen zweiten elektrischen Leiter, der sich von dem Antriebsgehäuse erstreckt und mit dem zweiten elektrischen Anschluss gekoppelt ist, um der zweiten Elektrode elektrische Energie bereitzustellen.

14. Endeffektor nach Anspruch 8 oder chirurgisches Werkzeug nach Anspruch 13, wobei jeder elektrische Anschluss einen im Allgemeinen U-förmigen Durchgang derart definiert, dass jede Elektrode sowohl ab das distale als auch das proximale Ende angeschlossen ist, und wobei das Messer in den Messerschlitz (418) durch Erstrecken mindestens teilweise durch den U-förmigen Durchgang jedes elektrischen Anschlusses hindurch erstreckbar ist.

15. Endeffektor nach Anspruch 8 oder chirurgisches Werkzeug nach Anspruch 13 oder 14, wobei sich der erste und der zweite elektrische Anschluss von den entsprechenden ebenen Dichtungsoberflächen der ersten beziehungsweise der zweiten Elektrode (414a, 414b) derart weg erstrecken, dass zwischen dem ersten und dem zweiten elektrischen Anschluss ein Spalt definiert wird, der das Messer aufnimmt.

## Revendications

1. Effecteur terminal, comprenant :
une première mâchoire (210) ayant une première électrode (414a) et un premier isolateur (502a) fixé à celle-ci ;
une seconde mâchoire (212) ayant une seconde électrode (414b) et un second isolateur (502b) fixé à celle-ci, les première et seconde mâchoires (210, 212) pouvant pivoter entre des positions ouverte et fermée ; et
une fente de couteau (418) définie coopérativement dans les première et seconde mâchoires (210, 212) et définissant une coupe transversale en forme de losange lorsque les première et seconde mâchoires sont dans la position fermée,
dans lequel chaque électrode fournit une étendue latérale interne (514) qui définit coopérativement la coupe transversale en forme de losange, et
dans lequel une longueur d'au moins l'une des étendues latérales internes (514) est supérieure à une épaisseur d'une correspondante parmi les première ou seconde électrodes (414a, 414b).

2. Effecteur terminal selon la revendication 1, dans lequel le premier isolateur (502a) et la première électrode (414a) définissent coopérativement une première partie de fente (504a) qui divise une première surface plane de la première électrode en premières parties gauche et droite (508a, 508b), et le second isolateur et la seconde électrode définissent coopérativement une seconde partie de fente (504b) qui divise une seconde surface plane de la seconde électrode en secondes parties gauche et droite (510a, 510b), et
dans lequel les première et seconde parties de fente (504a, 504b) définissent coopérativement la fente de couteau lorsque les première et seconde mâchoires sont dans la position fermée.

3. Effecteur terminal selon la revendication 2, dans lequel les premières parties gauche et droite (508a, 508b) fournissent chacune une étendue latérale externe (512) s'étendant à l'écart de la première surface plane et intégrée dans le premier isolateur, et les secondes parties gauche et droite fournissent chacune une étendue latérale externe s'étendant à l'écart de la seconde surface plane et intégrée dans le second isolateur, facultativement dans lequel chaque étendue latérale externe fournit un bord électriquement exposé qui fournit une transition entre l'étendue latérale externe et les première et seconde surfaces d'étanchéité planes.

4. Effecteur terminal selon la revendication 3, dans lequel les étendues latérales externes (512) sont intégrées dans les premier et second isolateurs de telle sorte que les premier et second isolateurs fournissent une interface affleurante avec les première et seconde surfaces d'étanchéité planes, respectivement.

5. Effecteur terminal selon l'une quelconque des revendications 2 à 4, dans lequel les premières parties gauche et droite (508a, 508b) fournissent chacune l'étendue latérale interne s'étendant à l'écart de la première surface plane, et les secondes parties gauche et droite (510a, 510b) fournissent chacune l'étendue latérale interne s'étendant à l'écart de la seconde surface plane, facultativement dans lequel les étendues latérales internes des premières et secondes parties gauche et droite définissent coopérativement la coupe transversale en forme de losange lorsque les première et seconde mâchoires sont dans la position fermée.

6. Effecteur terminal selon l'une quelconque revendication précédente, dans lequel chacun des isolateurs définit une section de rigole (516) s'étendant latéralement à travers la fente de couteau et reliant de ce fait structurellement des côtés latéraux des premier et second isolateurs.

7. Effecteur terminal selon l'une quelconque revendication précédente, dans lequel chaque électrode (414a, 414b) fournit un corps allongé avec des extrémités distale et proximale opposées, et un canal allongé est défini dans le corps et s'étend entre les extrémités distale et proximale pour former une partie de la fente de couteau.

8. Effecteur terminal selon la revendication 7, comprenant en outre :
un premier connecteur électrique (710) s'étendant à partir de l'extrémité proximale de la première électrode et conçu pour recevoir un premier conducteur électrique pour fournir de l'énergie électrique à la première électrode ; et
un second connecteur électrique s'étendant à partir de l'extrémité proximale de la seconde électrode et conçu pour recevoir un second conducteur électrique pour fournir de l'énergie électrique à la seconde électrode.

9. Outil chirurgical, comprenant :
un logement d'entraînement (208) ;
une tige allongée (202) qui s'étend à partir du logement d'entraînement ;
l'effecteur terminal selon la revendication 1 ;
une biellette d'entraînement (420) s'étendant à partir du logement d'entraînement et se terminant au niveau de l'effecteur terminal ; et
un couteau (416) accouplé fonctionnellement à la biellette d'entraînement et pouvant s'étendre dans la fente de couteau, dans lequel la biellette d'entraînement est actionnable pour déplacer longitudinalement le couteau au sein de la fente de couteau.

10. Outil chirurgical selon la revendication 9, dans lequel le premier isolateur et la première électrode (414a) définissent coopérativement une première partie de fente (504a) qui divise une première surface plane de la première électrode, et le second isolateur et la seconde électrode définissent coopérativement une seconde partie de fente (504b) qui divise une seconde surface plane de la seconde électrode, et
dans lequel les première et seconde parties de fente définissent coopérativement la fente de couteau lorsque les première et seconde mâchoires sont dans la position fermée, facultativement dans lequel la première surface plane fournit des étendues latérales externes opposées s'étendant à l'écart de la première surface plane et intégrées dans le premier isolateur, et la seconde surface plane fournit des étendues latérales externes opposées s'étendant à l'écart de la seconde surface plane et intégrées dans le second isolateur.

11. Outil chirurgical selon la revendication 10, dans lequel l'étendue latérale interne de chaque électrode est fournie par les première et seconde surfaces planes et chaque étendue latérale interne s'étend à l'écart des première et seconde surfaces planes, respectivement.

12. Outil chirurgical selon l'une quelconque des revendications 9 à 11, dans lequel chaque électrode fournit un corps allongé avec des extrémités distale et proximale opposées, et un canal allongé est défini dans le corps et s'étend entre les extrémités distale et proximale pour former une partie de la fente de couteau (418).

13. Outil chirurgical selon la revendication 12, comprenant en outre :
un premier connecteur électrique s'étendant à partir de l'extrémité proximale de la première électrode ;
un premier conducteur électrique s'étendant à partir du logement d'entraînement et accouplé au premier connecteur électrique pour fournir de l'énergie électrique à la première électrode ;
un second connecteur électrique s'étendant à partir de l'extrémité proximale de la seconde électrode ; et
un second conducteur électrique s'étendant à partir du logement d'entraînement et accouplé au second connecteur électrique pour fournir de l'énergie électrique à la seconde électrode.

14. Effecteur terminal selon la revendication 8 ou outil chirurgical selon la revendication 13, dans lequel chaque connecteur électrique définit un passage généralement en forme de U de telle sorte que chaque électrode est connectée à la fois aux extrémités distale et proximale, et dans lequel le couteau peut s'étendre dans la fente de couteau (418) en s'étendant au moins partiellement à travers le passage en forme de U de chaque connecteur électrique.

15. Effecteur terminal selon la revendication 8 ou outil chirurgical selon la revendication 13 ou la revendication 14, dans lequel les premier et second connecteurs électriques s'étendent à l'écart de surfaces d'étanchéité planes correspondantes des première et seconde électrodes (414a, 414b), respectivement, de telle sorte qu'un espace est défini entre les premier et second connecteurs électriques, lequel accueille le couteau.
